(19) **Europäisches Patentamt / European Patent Office / Office européen des brevets**

(11) **EP 2 658 581 B1**

(12) # EUROPEAN PATENT SPECIFICATION

(45) Date of publication and mention of the grant of the patent:
**30.05.2018 Bulletin 2018/22**

(51) Int Cl.:
*A61K 51/08* (2006.01)        *A61P 35/00* (2006.01)
*A61K 103/20* (2006.01)        *A61K 103/30* (2006.01)
*A61K 103/32* (2006.01)

(21) Application number: **11810808.3**

(22) Date of filing: **23.12.2011**

(86) International application number:
**PCT/EP2011/006558**

(87) International publication number:
**WO 2012/089336 (05.07.2012 Gazette 2012/27)**

(54) **A CONJUGATE OF HUMAN ALBUMIN AND 2-(4-ISOTHIOCYANATOBENZYL)-1,4,7,10-TETRAAZACYCLODODECANE-1,4,7,10-TETRAACETIC ACID USEFUL FOR THE LOCALIZATION OF RADIONUCLIDES FOR DIAGNOSTIC AND THERAPEUTIC PURPOSES**

KONJUGAT AUS MENSCHLICHEM ALBUMIN UND 2- (4-ISOTHIOCYANATOBENZYL) -1,4,7,10-TETRAAZACYCLODODECAN-1,4,7,10-TETRAESSIGSÄURE ZUR LOKALISIERUNG VON RADIONUKLIDEN FÜR DIAGNOSTISCHE UND THERAPEUTISCHE ZWECKE

CONJUGUÉ D'ALBUMINE HUMAINE ET D'ACIDE 2-(4-ISOTHIOCYANATOBENZYL)-1,4,7,10-TÉTRAAZACYCLODODÉCANE-1,4,7,10-TÉTRA ACÉTIQUE UTILE POUR LA LOCALISATION DE RADIONUCLÉIDES POUR DES FINS DIAGNOSTIQUES ET THÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2010 EP 10197071**

(43) Date of publication of application:
**06.11.2013 Bulletin 2013/45**

(73) Proprietors:
- **Universita' degli Studi di Genova
16129 Genova (IT)**
- **Istituto Europeo di Oncologia S.r.l.
20121 Milano (IT)**

(72) Inventors:
- **CAVIGLIOLI, Gabriele
I-16147 Genova (IT)**
- **PARODI, Brunella
I-16167 Genova (IT)**
- **CAFAGGI, Sergio
I-16121 Genova (IT)**
- **RUSSO, Eleonora
I-16147 Genova (IT)**

- **CIRRINCIONE, Paola
I-16148 Genova (IT)**
- **CHINOL, Marco
I-20141 Milan (IT)**
- **PAGANELLI, Giovanni
I-20141 Milan (IT)**

(74) Representative: **Ferreccio, Rinaldo
Botti & Ferrari S.r.l.
Via Cappellini, 11
20124 Milano (IT)**

(56) References cited:
**US-A1- 2003 059 368**

- **FOWLER J CHARLOTTE ET AL: "Dual-isotope lymphoscintigraphy using albumin nanocolloid differentially labeled with In-111 and Tc-99m", ACTA ONCOLOGICA (STOCKHOLM), vol. 46, no. 1, 2007, pages 105-110, XP002631492, ISSN: 0284-186X**

**(Cont. next page)**

- SCHILLER ET AL: "Yttrium-86-labelled human serum albumin microspheres: relation of surface structure with in vivo stability", NUCLEAR MEDICINE AND BIOLOGY, ELSEVIER, NY, US, vol. 35, no. 2, 20 December 2007 (2007-12-20), pages 227-232, XP022460571, ISSN: 0969-8051 cited in the application
- VAN DER PLOEG ET AL: "'Radioguided occult lesion localisation' (ROLL) for nonpalpable breast lesions: A review of the relevant literature", EUROPEAN JOURNAL OF SURGICAL ONCOLOGY, LONDON, GB, vol. 34, no. 1, 21 December 2007 (2007-12-21), pages 1-5, XP022392778, ISSN: 0748-7983 cited in the application
- JAKUBOWSKI NORBERT ET AL: "Labelling of proteins with 2-(4-isothiocyanatobenzyl)-1,4,7,10-tetraazacyclododecane-1,4,7,10-tetraacetic acid and lanthanides and detection by ICP-MS", JOURNAL OF ANALYTICAL ATOMIC SPECTROMETRY, vol. 23, no. 11, 2008, pages 1497-1507, XP008135186, ISSN: 0267-9477 cited in the application
- POVOSKI STEPHEN R ET AL: "A comprehensive overview of radioguided surgery using gamma detection probe technology", WORLD JOURNAL OF SURGICAL ONCOLOGY, BIOMED CENTRAL, LONDON, GB, vol. 7, no. 1, 27 January 2009 (2009-01-27), page 11, XP021050393, ISSN: 1477-7819, DOI: DOI:10.1186/1477-7819-7-11 cited in the application
- BERHO C ET AL: "Study of UV-vis responses of mineral suspensions in water", COLLOIDS AND SURFACES A: PHYSIOCHEMICAL AND ENGINEERING ASPECTS, ELSEVIER, AMSTERDAM, NL, vol. 248, no. 1-3, 9 November 2004 (2004-11-09), pages 9-16, XP004917347, ISSN: 0927-7757, DOI: 10.1016/J.COLSURFA.2004.08.046

**Description**

Field of application

The present invention is directed to the field of pharmaceutical and diagnostic industry and refers to a microdispersed system insoluble in aqueous environment, for the localization of radionuclides for diagnostic and therapeutic purposes and to a process for its preparation.

More particularly, the invention refers to a conjugate of human albumin (HA) and 2-(4-isothiocyanatobenzyl)-1,4,7, 10-tetraazacyclododecane-1,4,7, 10-tetraacetic acid (p-SCN-Bn-DOTA) insoluble in aqueous environment at pH values within 3 and 8.5, able to complex radionuclides, useful for diagnostic and therapeutic purposes, in particular for the treatment of mammalian neoplasias.

Known art

Over the last 20 years, the identification of mammalian neoplastic lesions at an earlier and earlier stage has allowed the development of more and more conservative surgical treatments.

Until few years ago the diagnosis of a non-invasive mammalian carcinoma did not exceed the 5% of cases. In the last decade the diffusion on a large scale of instrumental investigations, in particular of mammography and ultrasound, has allowed an increase in the identification of clinically hidden tumours (not perceptible by touch at the time of physical examination), which are histologically non-invasive in a percentage close to 25-30%. The objective of obtaining a precise pre-surgery localization of the lesion and a complete surgical removal has been the stimulus that has led researchers of the Istituto Europeo di Oncologia to develop a new technique for the localization and surgical treatment of nonpalpable mammalian lesions, which combines the use of radioactive tracers with the use of the probe for radioguided surgery (Paganelli G., Luini A., Veronesi U. et al. 2000. Use of technetium-99m-labeled colloid albumin for preoperative and intraoperative localization of nonpalpable breast lesions. Journal of the American College of Surgeons 190(6):692-699; Paganelli G., Veronesi U. 2002. Innovation in early breast cancer surgery: radioguided occult lesion localization and sentinel node biopsy. Nucl Med Commun 23(7):625-627).

This methodology for the localization of nonpalpable mammalian lesions, called R.O.L.L., English acronym for "Radioguided Occult Lesion Localization", includes the introduction of a suspension of macroaggregates of human albumin labelled with radioactive technetium ($^{99m}$Tc) into a group of microcalcifications or a small clinically nonpalpable nodule. By means of the probe for radioguided surgery, these lesions, frequently of tumoral nature, can be surgically removed in a targeted way maintaining the integrity of the breast.

The procedure of inoculation includes the use of macroaggregates of human albumin (MAA) of variable 10-100 $\mu$m diameter, labelled with $^{99m}$Tc with a specific activity of about 1700 MBq/mg. The day preceding the surgical procedure 10-15 MBq of radiolabelled drug are suspended in 0.2 ml of saline and injected, guided by ultrasound, into the centre of an opacity or, guided by the mammographic stereotaxic system, of a small group of microcalcifications. The verification of the correct localization can be obtained introducing into the lesion, immediately after the radioactive substance, a minimum amount of radiopaque solution. For a further verification, the so treated patient undergoes a scintigraphic investigation of the breast. The overlay of the image acquired by the gamma chamber, properly enlarged, and the mammographic one allows to confirm that the site of injection exactly corresponds to the site of the lesion.

The validity of this methodology is demonstrated by its diffusion also shown in various scientific reports (van der Ploeg C. et al. 2008. 'Radioguided occult lesion localisation' (ROLL) for nonpalpable breast lesions: A review of the relevant literature. EJSO 34(1):1-5; Povoski P., et al. 2009. A comprehensive overview of radioguided surgery using gamma detection probe technology. World J Surg Oncol 7).

The currently used radiolabelled drug (Technetium-MAA) does not allow, due to the short half-life of the radioactive decay ($t_{1/2}$= 6.02 hours) of the 99 radioisotope of technetium ($^{99m}$Tc), a planning of surgical procedures able to address the growing request of patients with lesions that should undergo radioisotopic localization.

Hence, the use of a radiolabelled drug with a longer half-life would allow to plan surgical procedures on several days and, not negligible aspect, also to carry out the localization as an outpatient exam as well as its verifications in the days preceding surgery, further reducing hospitalization times and costs.

The radiolabelled drug used up to date is an aspecific complex between macroaggregates of human albumin (MAA) and $^{99m}$Tc that is prepared at the time of use through different marketed kits from various manufacturers: Bracco, CIS-US, Draximage, GE Healthcare and Mallinckrodt. These kits differentiate for the amount of aggregated human albumin, SnCl$_2$, and preservatives they contain. They are provided to the centres of nuclear medicine as freeze-dried powders of the non-radioactive ingredients, preserved under a nitrogen atmosphere. The operator adds 40-100 mCi of sodium pertechnetate (Na$^{99m}$TcO$_4$) to obtain the final radiolabelled drug. After stabilization at room temperature for 15 min, in order to ensure the maximum labelling of $^{99m}$Tc to human albumin (HA), the kit can be diluted with isotonic sterile solution of 0.9% NaCl (w/v) (or solution of sodium chloride for parenteral preparations (FU XII), hereinafter 0.9% NaCl) and injected into the patient.

The soluble form of $^{99m}Tc$, i.e. sodium pertechnetate ($Na^{99m}TcO_4$), is obtained from $99Mo/^{99m}T_c$ generators, by elution with saline.

The operator adds to the freeze-dried MAA a volume of few milliliters of $Na^{99m}TcO_4$ in injectable saline, depending on the radioactivity that is intended to achieve.

$SnCl_2$ is a strong reducing agent contained in MAAs themselves that, put in contact with the weak oxidizing agent $Na^{99m}TcO_4$, allows complete conversion of the soluble form of $^{99m}Tc$ into the $Tc^{+3}$ ion that precipitates as $TcCl_3$ within the MAAs. The strength and amount of the reducing agent are such to ensure complete reduction of $Na^{99m}TcO_4$ avoiding the need to remove residual $Na^{99m}TcO_4$.

Sn(II) must be converted into the water-soluble $SnCl_4$ but, if conditions are not carefully controlled, it can convert, in aqueous environment and in the presence of oxygen, also in Sn(OH)Cl, poorly soluble in aqueous environment. The ATC code for this radiolabelled drug is V09EB01 (Technetium particles for injection (Macrosalb)).

A typical formulation of freeze-dried MAAs (MAASOL GE Healthcare) contains:

1.75 mg MAA/vial

Stannous Chloride dihydrate 0.175 mg/vial

Sodium acetate

Poloxamer 238

$N_2$ atmosphere

Sodium acetate in solution will form the buffering system at pH 4.8-5.8, pH at which MAAs form and stabilize. Poloxamer is an adjuvant that aids the redispersion of the freeze-dried product in the injection medium keeping the size of the aggregates smaller than 100 $\mu$m. The nitrogen atmosphere is necessary to prevent the oxidation of stannous chloride by atmospheric oxygen.

The main diagnostic indication for which MAAs are licensed after labelling with $^{99m}Tc$ is pulmonary perfusion scintigraphy, as a secondary indication they are used in venous scintigraphy and in some cases they are also indicated for the evaluation of the peritoneal-venous shunt after intraperitoneal administration.

Additionally, it should be reminded that the Italian Drug Agency (AIFA) recently released an alert [Information Note of March 2009 concerning radiolabelled drugs containing albumin (Maasol, Macrotec, Nanocoll, Pulmocis, Technescan Lyomaa, Ventricoll): safety information.] concerning the reporting of anaphylactic adverse reactions with lethal outcome associated to intravascular administration of radiolabelled drugs containing HA.

Since a causal relationship between the adverse reaction and MAA could not be established with certainty, it is legitimate to suspect also other components of the preparation, not least the Sn compounds.

The aspecificity of the association between Tc and MAA could also be the cause of the phenomenon, however recorded as not very frequent, of diffusion and/or drainage of the radionuclide in healthy tissues and/or in the lymphatic circulation.

These latter considerations, associated with the primary need of finding a radiolabelled drug with a half-life longer than the 6.02 hours of $^{99m}Tc$, represent the rationale that has led to the achievement of the invention disclosed herein.

The macroaggregates of HA (MAA) are obtained through different procedures that differentiate for some details and have given rise to as many patents.

In 1969 Bowen et al. (Preparation of Technetium-99m Human Serum Albumin Macroaggregates, Am J of Hospital Pharm., 26:529-534, 1969) had already provided a detailed description of these macroaggregates.

When referring to MAA one should refer to particles of HA insoluble in aqueous phase, with a range of dimensional distribution between 3 and 200 $\mu$m that are reduced to 10 to 100 $\mu$m for therapeutic use. The selection of the particle size is carried out by filtrations through controlled porosity membranes, to exclude particles with larger sizes, and by centrifugation in order to remove particles with smaller sizes not readily sedimented.

The various procedures for the preparation of MAAs include:

1. denaturation of HA by exposure to extreme pHs, for example pH 1-2 by using strong acids (sulphuric acid, hydrochloric acid), to heat, bases or organic solvents;

2. addition of a suitable amount of $SnCl_2$ in solution stabilized with HCl;

3. adjustment of pH in the range between 4 and 6, generally with an acetate buffer, in the proximity of the isoelectric point of denatured HA (about 5.3), that determines an initial precipitation of colloidal aggregates;

4. subsequent heating between 50 and 90°C, but in some cases 110°C are also reached, that determines the formation of MAAs and their stabilization. These heating modalities, which influence the dimensional distribution of the aggregates, are obtained in the most different ways also alterning cycles of heating and quick cooling.

MAAs, dimensionally selected by filtration and centrifugation, can then be freeze-dried with the addition of suitable adjuvants: anti-aggregating, antifoam agents, preservatives, or not denatured HA to facilitate reconstitution.

Sterility and apyrogenicity of the final product is guaranteed by the sterility and apyrogenicity of the used materials and by the control of environmental conditions (US 4,024,233; US 4,094,965).

In a recent patent (US 6,730,286), inter alia, an improvement in the production of these MAAs through preventive purification of the solution of HA by ultrafiltration is underlined.

From the analysis of preceding patents and publications it is clear that the formation of MAAs is a process requiring several preparative steps.

The direct labelling of MAA with $^{111}$In, as it has been demonstrated by Watanabe N. et al. (Journal of Nuclear Medicine 38(10):1590-1592, 1997), produced a stability of the MAA-radioisotope complex as short as 180 minutes.

This result suggested to conjugate to MAAs complexes able to bind radioisotopes for a longer time.

Then, at first the Applicants investigated the possibility to carry out a conjugation reaction with p-SCN-Bn-DOTA directly on MAAs. Such reaction produced poor results in terms of conjugation and of reproducibility. In fact, the formation of MAAs is based on the interactions between the protein amine and carboxylic groups, and free amine groups are present on the surface of MAAs in minimal and variable amounts or they are even absent.

As reported by Patil (Drug Dev. Res. 58:219-247, 2003) HA was also transformed into micro- and nanospheres that could be used for diagnostic and therapeutic purposes. It is clear that the microspheres (MS) can be an interesting substrate for preparing a carrier for radiolabelled drugs.

The formation of microspheres involves complex multi-step procedures, and the use of oily phases, organic solvents, and reticulating agents such as glutaraldehyde. For example, a preparative modality involves emulsifying an HA aqueous solution in oil (e.g. cottonseed oil), the emulsion W/O is heated to 110°C or more until microspheres are formed. MS are separated by filtration and washed with solvents (e.g. acetone, ether, heptane).

Another preparative mode involves pouring an aqueous solution of HA in anhydrous ethanol at -15°C. Microspheres are then hardened with glutaraldehyde. The reaction is quenched with metabisulfite. Eventually microspheres are filtered and washed with abundant water.

It is to consider anyway that the architecture of MS involves an interaction between carboxylic and amine groups of the protein, or the consumption of these amine groups for the cross-linking reaction with a reticulating agent such as glutaraldehyde.

On the basis of these considerations it is clear that the formation of microspheres of HA (MSHA) involves a complex preparation with several preparative steps characterized by the wide use of non aqueous solvents.

Schiller et al. (Nuclear Medicine and Biology 35(2):227-232, 2008) studied the labelling with $^{86}$Y of microspheres of HA conjugated with DOTA. Such conjugation was obtained using p-SCN-Bn-DOTA. Such microspheres designed for the treatment of liver neoplasias were assayed by injecting, in the caudal vein of Wistar rats, 50-100 $\mu$g of MS (2 MBq). Microspheres having a diameter between 20 and 30 $\mu$m were obtained by heating (100-130°C) an aqueous solution of HA emulsified with olive oil (according to the specifications of the manufacturer cited by Schiller et al. (ROTOP Pharmaka AG) for HSA Mikrosphaeren B20, 3.251 mg powder contain, as the active substance, 2.5 mg denatured human albumin (300,000 -500,000 microspheres, ø 10-30 $\mu$m) and, as excipients, stannous chloride dihydrate, polysorbate 80, bengal rose disodium and nitrogen). MS had a specific surface of 0.26 m$^2$/g and a conjugation ratio of $2 \times 10^{-7}$ moles per milligram of microspheres. Conjugation occurred using a p-SCN-Bn-DOTA:MSHA molar ratio of 100:1 in aqueous phase at pH 9 using $Na_2B_4O_7$ as a buffer.

Using the same reaction conditions but using HA as a substrate, conjugation does not occur, probably because the protein undergoes partial hydrolysis due to the excessively high pH.

Jakubowski et al. (J. Anal. At Spectrom. 23(11):1497-1507, 2008) describe a conjugation procedure of bovine serum albumin (BSA) by p-SCN-Bn-DOTA in carbonate buffer at pH 9 at 20°C, using 6 nmol of BSA, an optimized reaction molar ratio p-SCN-Bn-DOTA/BSA of 40/1 and a conjugation reaction time between 16 and 24 hours. The conjugation product DOTA-BSA obtained by these authors is soluble in the aqueous medium of reaction. In fact, the solubility of their product in acetate buffer (pH 7-7.5; 0.5M) also allows the elution of the conjugate in the subsequent chromatographic purification procedure described by the authors. Always in aqueous solution and in the presence of acetate buffer, i.e. in homogeneous phase, the conjugation product is labelled with Europium, with a reaction molar ratio of Eu/p-SCN-Bn-DOTA of 10/1, using a labelling temperature of 37°C for 30 min. The maximum conjugation molar ratio, estimated by measuring the TXRF (total reflection X-ray fluorescence) of Eu in the protein, conjugated and labelled, is 4.1 Eu/BSA.

US 2003/0059368 discloses, in example 50, the labeling of albumin with Samarium. This is obtained by at first reacting p-SCN-Bn-DOTA with samarium chloride in water and then adding the resulting solution to albumin dissolved in carbonate buffer at pH 9, followed by mixing at 25°C for 60 minutes. The final solution was dialyzed and lyophilyzed to obtain a solid containing 10 mol of SM per mol of albumin.

The product of example 50, as stated at par. 0236 and 0329 for all the products of examples 38 to 51, is for use in determining glomerular flow rate (GFR) and glomerular integrity rate. Thus solubility is a mandatory requirement for a product that must reach the renal glomerulus and, in fact, in par. 0083 it is stated that the compounds of the invention are administered as physiologically compatible solutions within a pH range of about 5 to about 9. Thus the complex of example 50 is soluble in water at this pH range.

Fowler J. C. et al., Acta Oncologica (Stockholm), vol. 46, no. 1, 2007, pages 105-110, discloses a conjugate of human albumin nanocolloid to p-SCN-Bn-DOTA, which complexes $^{111}$I or $^{99m}$Tc, for use in sentinel lymph node identification. In order to be used for such a purpose, it must have a size lower than 100 nm, because otherwise it would not be drained from the tissues into the limphatic system. As a matter of fact, by virtue of its nano-size, this conjugate forms pseudosolutions or colloidal solutions, which allow its purification by means of gel filtration on Sephadex G-25 PD10 at the pH of a physiological solution (4.5-7.0) (page 106, right column). The eluted fractions show an absorbance (measured by a transmittance detector) at 298 nm right because the conjugate forms a colloidal solution.

The starting nanocolloid used by Fowler J. C. et al. contains, further to human albumin, stannous chloride dihydrate, anhydrous glucose, poloxamer 238, sodium phosphate, dibasic, anhydrous and sodium phytate anhydrous.

Summary of the invention

In one aspect, the present invention relates to a conjugate of human albumin (HA) and 2-(4-isothiocyanatobenzyl)-1,4,7,10-tetraazacyclododecane-1,4,7,10-tetraacetic acid (p-SCN-Bn-DOTA) insoluble in aqueous medium at a pH of 3-8.5 and whose IR spectrum shows an absorption at about 700 cm$^{-1}$.

HA, as intended hereinafter, means human albumin.

In a further aspect, the present invention relates to a conjugate of HA and p-SCN-Bn-DOTA insoluble in aqueous medium at a pH of 4-8 and whose IR spectrum shows an absorption at about 700 cm$^{-1}$.

In another aspect, the present invention relates to a complex consisting of said conjugate of HA and p-SCN-Bn-DOTA (HAC) and of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu. In particular the radioactive isotope is selected among the group consisting of $^{111}$In, $^{177}$Lu, $^{90}$Y, $^{67}$Cu, $^{64}$Cu and radioactive lanthanides and is preferably $^{177}$Lu.

In particular, among the radioactive lanthanides, $^{140}$La, $^{149}$Pm, $^{153}$Sm, $^{152}$Tb, $^{166}$Ho, $^{169}$Er and $^{175}$Yb are cited.

The above mentioned complex can be used for in vivo localization of radionuclides in organs or tissues of the human body for diagnostic or therapeutic purpose and in particular for the localization of mammalian neoplastic lesions by the R.O.L.L. methodology ("Radio guided Occult Lesion Localization").

According to another aspect, the invention relates to a process for preparing a conjugate of HA and 2-(4-isothiocyanatobenzyl)-1,4,7,10-tetraazacyclododecane-1,4,7,10-tetraacetic acid (p-SCN-Bn-DOTA), comprising the steps of:

a) reacting HA with p-SCN-Bn-DOTA in aqueous medium at a temperature between 35°C and 45°C, with the formation of a suspension, containing a solid phase suspended in a liquid phase;

b) separating said solid phase, containing said conjugate, from said liquid phase, possibly containing unreacted p-SCN-Bn-DOTA.

Said separation step b) can be a filtration step or, preferably, an ultrafiltration step.

The solid obtained from the filtration step, as well as the retentate from the ultrafiltration step, can be subjected to freeze-drying.

The reaction between HA and p-SCN-Bn-DOTA is carried out preferably at a temperature of 38-42°C for 4-24 hours, preferably for 8-21 hours.

The aqueous medium in which said reaction occurs is preferably an aqueous solution with ionic strength equal to 0.1-0.5M, in particular a 0.9% NaCl solution, optionally buffered at pH 4-8.5.

In the ultrafiltration step a membrane having a cut-off of 10 kDa is generally used.

In the reaction between p-SCN-Bn-DOTA and HA, p-SCN-Bn-DOTA and HA are typically used in a molar ratio between 100:1 and 1:1, preferably between 30:1 and 16:1.

Preferably HA subjected to the reaction step a) with p-SCN-Bn-DOTA is previously purified by ultrafiltration or dialysis with a membrane having a cut-off of 10 kDa, in the presence of a solution of ionic strength 0.1-0.5M, in order to remove the thermal stabilizers present in commercially available HA, in particular sodium caprylate and sodium acetyl tryptophanate.

In a further aspect, the present invention refers to a conjugate of human albumin (HA) and 2-(4-isothiocyanatobenzyl)-1,4,7,10-tetraazacyclododecane-1,4,7,10-tetraacetic acid (p-SCN-Bn-DOTA) insoluble in aqueous medium at a pH of 3-8.5 and whose IR spectrum shows an absorption at about 700 cm$^{-1}$, obtainable by the above described process.

In a further aspect, the present invention refers to a process for preparing a complex as described above, comprising the steps of:

a) dispersing a conjugate as described above in aqueous buffer at a pH of 4-6;

b) adding a water-soluble compound containing an ion of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu and heating under stirring at a temperature of 30-90°C for 20-40 minutes.

The water-soluble compound added to the dispersion from step a) is, in a particular aspect of the present invention, $^{177}LuCl_3$.

As an aqueous buffer in step b) acetic acid/acetate buffer is preferably used, in particular 1M with a pH of 5.

In a further aspect, the present invention refers to a kit for preparing a complex as described above, including a conjugate of HA and p-SCN-Bn-DOTA and a water-soluble compound containing an ion of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu.

Said water-soluble compound is preferably $^{177}LuCl_3$.

In the kit according to the invention, said conjugate is conveniently in a lyophilized form and said water-soluble compound is in the form of an aqueous solution.

The conjugate according to the present invention allows to obtain complexes with stable radioisotopes, suitable in particular for the localization of mammalian neoplastic lesions, in particular with the R.O.L.L. methodology. The above mentioned complexes, insoluble in aqueous medium, are not subjected to diffusion or drainage phenomena of the radionuclide in healthy tissues and/or in the lymphatic circulation and are instead provided of a half-life that allows to plan possible surgical procedures, following the diagnostic results, over several days.

Further advantages and characteristics of the present invention will result from the following detailed description, in which reference is made to some figures, as described hereinafter.

Short description of the figures

Figure 1 shows the variation of the degree of substitution (DS) of p-SCN-Bn-DOTA in the HAC conjugate upon varying the concentration of carbonate buffer.

Figure 2 shows a comparison between the gelatinous precipitate obtained by the process according to the present invention (right microtube) and the solution deriving from treatment of HAP with carbonate buffer and DOTA for 20 h at 40°C (left microtube).

Figure 2A shows the formation of a gelatinous precipitate in the first hour of reaction of the process according to the invention.

Figure 3 shows a comparison between the IR spectrum of the freeze-dried conjugate according to the invention (top) and the IR spectrum of purified albumin (HAP) (bottom) subjected to the same reaction conditions used in the preparation of the conjugate according to the invention, using DOTA instead of p-SCN-Bn-DOTA.

Figure 4 is a picture displaying a comparison between the freeze-dried conjugate according to the invention (indicated with C) and purified freeze-dried albumin (HAP).

Figure 5 shows a comparison between DSC profiles of the conjugate according to the invention (full line) and HAP (broken line).

Figure 5A shows DSC profiles of a freeze-dried sample of purified albumin (HAP) pre-treated at 100°C (full line) and subjected to a subsequent gradient from -20°C to 250°C (broken line).

Figure 5B shows DSC profiles of a freeze-dried sample of a conjugate according to the invention pre-treated at 100°C (full line) and subjected to subsequent gradient from -20°C to 250°C (broken line).

Figure 6 is a picture displaying a comparison between residual samples from the DSC study of Figure 5, wherein the residue of the conjugate according to the invention is the one on the right side.

Figure 7 is a micrograph of a sample of a conjugate according to the present invention in colloidal suspension in 1M sodium acetate pH 5 subjected to prolonged (20 h) heating (40°C).

Figure 8 is a graph reporting a calibration curve (absorbance vs. $\mu$moles) built on the basis of the data reported in the Table 3 of Example 1.

Figure 9 is a graph reporting a calibration curve (emission at 261.542 nm vs. $\mu$g/ml) built on the basis of data reported in the Table 5 of Example 2.

Figure 10 displays an ITLC plate (instant thin-layer chromatography) carried out on HAC samples radiolabelled with $^{111}$In (1 mCi/mg), in order to evaluate its radiochemical purity.

Figure 11 displays an ITLC plate (instant thin-layer chromatography) carried out on HAC samples radiolabelled with $^{177}$Lu (1 mCi/mg), in order to evaluate its radiochemical purity.

Figure 12 displays an ITLC plate (instant thin-layer chromatography) carried out on HAC samples radiolabelled with $^{177}$Lu (2 mCi/mg) from another batch, in order to evaluate its radiochemical purity.

Figure 13 is a MALDI-TOF spectrum of purified human albumin.

Figure 14 is a comparison among the MALDI-TOF spectrum of purified human albumin (HAPP) and MALDI-TOF spectra of four samples of conjugate HAC accordng to the invention.

Figure 15 is a densitometric evaluation of ITLC of commercial HA and purified HA (HAP) (measurement of reflection in absorbance at 280 nm).

Figure 16 is a densitometric evaluation of ITLC of an HAC sample (measurement of reflection in absorbance at 280 nm).

Figure 17 shows two images obtained by overlapping the respective $\mu$-PET and $\mu$-CT images obtained from two rats injected with $^{64}$Cu-HAC.

Figure 18 is a graph reporting the uptake value for $^{64}$Cu-HAC into mammary glands of rats.

Detailed description

The present invention relates to a conjugate of HA and p-SCN-Bn-DOTA, hereinafter called HAC, insoluble in aqueous environment between pH 3 and pH 8.5, able to complex radionuclides, obtainable with a procedure including a single reactive step. The above mentioned conjugate represents an insoluble microdispersed system that can substitute MAAs because it can complex by coordination some radionuclides that can be used for diagnostic and therapeutic purposes. In fact, the conjugate according to the invention is able to stably complex various ions and thus can be labelled with different radioisotopes forming complexes with DOTA that are stable and kinetically inert.

DOTA, or 1,4,7,10-tetraazaciclododecane-1,4,7,10-tetraacetic acid, is a known macrocyclic ring chelating agent, giving complexes stable in time with various metals, with a two-step kinetics of incorporation of the metal in the macrocycle, consisting in the subsequent deprotonation of nitrogen atoms and carboxylic groups of the ring. This mechanism has a first pH-controlled quick step that involves only the nitrogen atoms on the ring, and a second slower step (step of cage closure), in which participate the carboxylic groups that give stability to the complex.

The conjugate according to the present invention contains the DOTA structural moiety, introduced in the HA molecule thanks to the reactivity of the isothiocyanate function of p-SCN-Bn-DOTA.

The degree of conjugation of p-SCN-Bn-DOTA in HAC is such to allow a suitable radioisotopic labelling for its application in the R.O.L.L. or in other similar diagnostic and therapeutic methodologies. Furthermore, differently from other protein substrates, such product resists to the labelling procedures that involve high temperatures, for example 90°C for 30 min, conditions necessary to effectively complex, in the DOTA macrocycle, lanthanides such as lutetium.

HAC is able to be inoculated in tissues, following radioisotopic labelling, as a colloidal precipitate or as a microdispersion, also derived from the reconstitution of freeze-dried samples, and there to exert its diagnostic and therapeutic activity for a time not shorter than the half-life of the radioactive decay of the radionuclide.

Among the useful radionuclides in the achievement of the present invention some gamma emitters can be cited, such as $^{111}$In, with a $t_{1/2}$ of 67 h (2.79 days) and $^{177}$Lu, with $t_{1/2}$ of 6.64 days. The latter is particularly interesting because it displays, beside the gamma emission necessary for imaging, a β- emission of intermediate energy (500 keV, maximum penetration 2 mm) very useful in the treatment of small size tumors. Furthermore, the energy of gamma photons emitted from $^{177}$Lu is very close to that of $^{99m}$Tc, the most widely used radioisotope in nuclear medicine, and then compatible with the currently used γ-chambers.

Among the other radionuclides that can be used in the present invention $^{90}$Y, and $^{67}$Cu ($t_{1/2}$ 2.58 days) can be cited. In particular, $^{90}$Y is considered by many the most important isotope in nuclear-medical therapy. Such radionuclide is a pure high energy β- (2.27 kEv) with maximum penetration in tissues exceeding 1 cm and $t_{1/2}$ of 64 hours (2.6 days). Since it lacks y emissions, it cannot be used in imaging or in dosimetry, though the great advantage of this radionuclide is that also the tumour cells more distant from the inoculation point, lying in a radius of 2 cm, can be hit; this effect, known as "crossfire effect", is considered important in the treatment of solid tumours.

Further directions about useful radionuclides for the present invention can be taken from "Radionuclide selection and model absorbed dose calculations for radiolabelled tumour associated antibodies" B. Wessels, R. Rogus. Med Phys. 11(5) 1984 pages 638-645, and from "Metallic radionuclides for radioimmunotherapy." A.R. Fritzberg, C.F. Meares in "Cancer radioimmunotherapy: Present and Future" Harwood Academic Publishers, Switzerland, Editore Riva P, pages 57-79, 1999.

In the following Table 1 a list is reported by way of indication, and not exhaustive, of the radionuclides that can be used in the present invention.

Table 1

| Radionuclide | $t_{1/2}$ | Decay (MeV) | Rmax |
|---|---|---|---|
| 67Cu | 2.58 d | β(0.54), γ(0.185) | 1.8 mm |
| 64Cu | 12.8 h | β(0.57), γ(0.081), β⁺(0.653), γ(0.511) | 2.5 mm |
| 89Sr | 50.5 d | β(1.49) | 8.0 mm |
| 90Y | 2.67 d | β(2.28) | 12.0 mm |
| 105Rh | 1.48 d | β(0.57), γ(0.320) | 1.9 mm |
| 109Pd | 13.6 h | β(1.00) | 4.6 mm |
| 111Ag | 7.47 d | β(1.05), γ(0.340) | 4.8 mm |
| 153Sm | 1.95 d | β(0.80), γ(0.103) | 3.0 mm |
| 169Er | 9.50 d | β(0.34) | 1.0 mm |
| 177Lu | 6.67 d | β(0.50), γ(0.208) | 1.5 mm |
| 111In | 67.4 h | γ(0.173), γ(0.247) | |
| 68Ga | 68 min | β⁺(1.90), γ(0.511) | |
| 166Ho | 26.8 h | β(1.85), γ(0.081) | 8.7 mm |
| 198Au | 2.70 d | β(0.97), γ(0.411) | 4.4 mm |
| 213Bi | 1.00 h | α(7.80), γ(0.720) | 70 μm |
| 72Zn | 46.5 h | β(0.45) | 1.5 mm |
| 65Ni | 2.52 h | β(2.14) | 10.0 mm |
| 165Dy | 2.3 h | β(1.3) | 7.0 mm |

The chelating agent used to form the conjugate according to the invention is 2-(4-isothiocyanatobenzyl)-1,4,7,10-tetraazacyclododecane-1,4,7,10-tetraacetic acid, also known as p-SCN-Bn-DOTA or p-SCN-Bz-DOTA.

The process to obtain the conjugate according to the invention includes the steps of:

a) reacting human albumin (HA) with p-SCN-Bn-DOTA in an aqueous medium at a temperature between 35°C and 45°C, with the formation of a suspension, containing a solid phase suspended in an aqueous phase;

b) separating the solid phase, containing the conjugate, from the liquid phase, possibly containing unreacted p-SCN-Bn-DOTA;

Said separation step b) can be a filtration step or, preferably, an ultrafiltration step.

The solid obtained from the filtration step, as well as the retentate of the ultrafiltration step, can undergo freeze-drying. The conjugation procedure, object of this invention, preferably includes the use of extremely pure HA, hereinafter called HAP.

HAP is obtained from HA samples of European Pharmacopoeia quality, properly purified eliminating thermal stabilizers, as for example sodium caprylate and N-acetyl triptophan, added during the process of extraction and purification from the haemoderivative.

In order to promote the reaction between isothiocyanate and the nucleophilic sites of HA, including amine groups, these sites must be freed from thermal stabilizer molecules.

In fact, the interaction between the carboxylic group of tryptophan and the amine sites of HA is well-known, from the observations made by McMenamy et al. (JBC 1958, 233, 6, 1436-1446).

The purification is carried out by repeatedly washing the HA solution with 0.9% NaCl on ultrafiltration membranes having a cut-off of 10 kDa.

The method according to the present invention uses as a substrate mainly HA or human albumin obtained through the technology of recombinant DNA. Valuable substrates of this reaction can be water-soluble HA derivatives or HA analogues such as water-soluble proteins having composition, molecular weight and isoelectric point similar to HA, provided that they are highly purified.

HA has been used in therapy for numerous years and is thus present on the market with a purity regulated by numerous pharmacopoeias.

For example, medicinal products based on HA in solution represent a source of high quality HA directly usable in humans. The conjugation reaction here described and the resulting reaction product, insoluble in aqueous environment, represent the present invention.

Medicinal solutions of HA contain variable amounts of thermal stabilizers, as in the case of solutions marketed by Baxter (Human Albumin Baxter), or by Alpha Therapeutic Italia SpA (Albutein) containing, in addition to 200 mg/ml of HA, 16 mmol/l (2.7 g/l) of sodium caprylate and 16 mmol/l (4.3 g/l) of sodium acetyl tryptophanate.

If HA containing a stabilizer is used, the first step of the process that leads to the insoluble conjugate according to the invention involves the purification of HA to remove, by using high ionic strength solutions, thermal stabilizers and other possibly present impurities. This purification phase is essential to allow to free the sites involved in conjugation with the isothiocyanate phenyl derivative of DOTA, then the reaction itself and its progress.

Such sites are represented by nucleophilic sites of the lateral chains of amino acids such as cysteine, lysine, histidine and arginine.

Purification can be obtained by ultrafiltration or dialysis on membranes having a cut-off equal to 10 kDa in the presence of a solution of suitable ionic strength (0.1-0.5M, specifically 0.154M).

The conclusion of the purification process can be evaluated by UV spectrophotometry, by the absence of the typical absorbance of sodium acetyl tryptophanate ($\lambda$max =280 nm) in the ultrafiltrate of the last washing. GC-MS studies on the ultrafiltrate, dried and reconstituted with a suitable amount of acid methanol warmed for some minutes, show that when such absorbance is no more spectrophotometrically detectable, also the gas chromatogram of the related residual does not show any presence neither of methyl octanoate nor of N-acetyl tryptophan-methyl derivative.

The solution of purified HA (HAP) in 0.9% NaCl can be used as such or freeze-dried and used after reconstitution. The purification and freeze-drying processes allow to obtain HAP yields higher than 98% (w/w) with respect to the HA content of the medicinal solution used. The concentration of HA in HAP is determined by spectrophotometric UV dosing at 280 nm versus a pure standard HA (Albumin from human, fatty acid free, globulin free, A3782 Sigma-Aldrich). The HAP solution is stored between 2°C and 6°C and shows to be reactive for long to the conjugation with p-SCN-Bn-DOTA.

Such reaction occurs with a molar ratio of conjugation p-SCN-Bn-DOTA/HAP ranging between 100/1 and 1/1; in particular, in the range 16/1 to 30/1 yields of HAC product higher than 80% (wHAC/wHAP %) are obtained.

The conjugation reaction is influenced by different parameters including: pH, buffer species, buffering power, ionic strength, in addition to temperature and reaction time.

The molar ratio of reaction influences, in addition to the yield of the reaction, the degree of substitution (DS= nDOTA/nHA) of p-SCN-Bn-DOTA in the HAC conjugate.

"Degree of substitution" (DS) means the number of molecules of p-SCN-Bn-DOTA that react with reactive groups of HA (amine, oxidrilic and thiolic groups) per number of molecules of HA. This also corresponds to the number of DOTA structural moieties introduced in each molecule of HA.

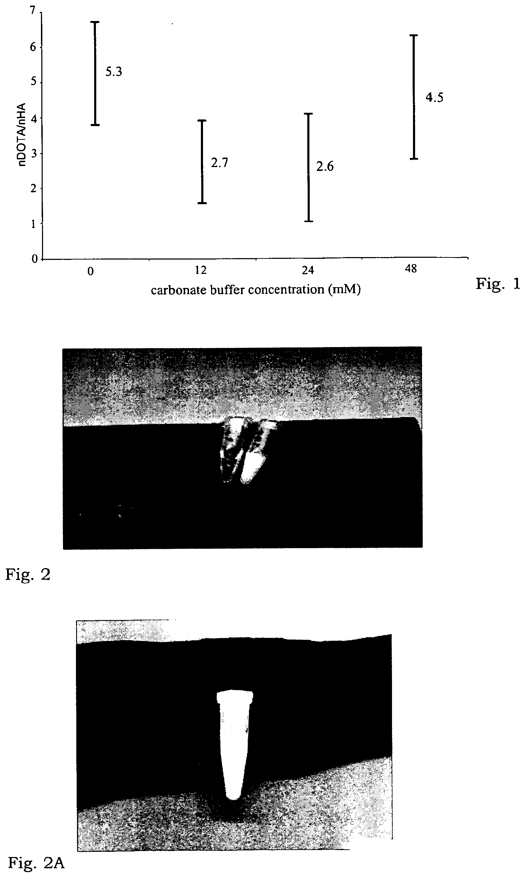

The reaction occurs between pH 4 and pH 8.5, and the pH of reaction influences the DS. In Fig. 1 the variation of the DS depending on the concentration of carbonate buffer in the reaction medium is reported. DS is expressed as a 95% confidence interval of the molar ratio given by the number of macrocyclic functions of p-SCN-Bn-DOTA added to one molecule of HA. The mean value is numerically indicated at the centre of the bar that represents the interval.

The presence or the absence of a buffer, such as for example a carbonate buffer or other efficient buffers in the above defined range of pH for the reaction, influences, also depending on the buffering power, the type of precipitation of HAC, which for this reason will appear in a more or less gelatinous form.

Controlling the temperature of reaction is essential, in fact below 35°C the reaction does not proceed in a significant way. As optimal range of temperature for the reaction, the range between 35°C and 45°C, and more specifically the range 40 $\pm$ 2°C can be defined.

The reaction time influences the progress of the reaction and then the DS of the conjugate. After few minutes at 40°C the formation of a white gelatinous precipitate is appreciated, indicating the formation of the conjugate (Fig. 2A). A good yield of HAC is obtained prolonging the reaction time between 4 and 24 hours, depending on the reagents concentrations, pH, buffer system used, buffering power and ionic strength. An optimal time of reaction seems to be between 8 and 21 hours.

The formation of a gelatinous precipitate during the first hour generates high viscosity in the reaction medium that strongly restricts the diffusion of the reagent p-SCN-Bn-DOTA and then the progress of the reaction itself.

To this regard we have observed that it is appropriate to counter this increase of viscosity by dilution, to promote a good progress of the reaction and to obtain a suitable product for the subsequent radioisotopic labelling. To this aim, two aliquots of 0.9% NaCl solution, each equal to 1/2 of the initial volume of the reaction medium, are added in two successive times, separated by 15 min, after the first hour of reaction.

The tactic of splitting the dilution in time allows to promote the diffusion of the reactive species without negatively influencing the reaction rate with a sudden decrease of p-SCN-Bn-DOTA concentration.

An alternative way to conduct the reaction with good yield and rate is that of starting the reaction by adding 1/3 of the total amount of p-SCN-Bn-DOTA to the HA solution and then adding another 1/3 of p-SCN-Bn-DOTA dissolved in a volume of 0.9% NaCl aqueous solution corresponding to 3/5 of the initial volume of HA solution after 1-1.5 h and the final 1/3 of p-SCN-Bn-DOTA dissolved in a volume of 0.9% NaCl aqueous solution corresponding to 3/5 of the initial volume of HA solution after 4-4.5 h.

A further alternative way to conduct the reaction with good yield and rate comprises the provision of a p-SCN-Bn-DOTA solution containing 12 mg/mL of p-SCN-Bn-DOTA in a 0.9% NaCl aqueous solution and the addition thereto of 50 mg of HAP (properly purified by repeated washing with deionized water and freeze-dried) per each mL of the above p-SCN-Bn-DOTA solution, wherein HAP is divided into 5 identical aliquots, which are added during 10-15 hours at intervals of 120-180 minutes.

Preferably, after the third and the last addition, the reaction medium is diuted with an amount of 0.9% NaCl solution corresponding to a half of the initial volume of the p-SCN-Bn-DOTA solution.

The ponderal mean yield of HAC samples (n=5), purified, freeze-dried and with DS in the range between 2 and 5 (nDOTA/nHA), calculated in comparison with the weight of HAP used in the reaction, varies in the range 87% $\pm$ 5% (wHAC/wHAP %).

The product HAC is characterized by its insolubility in aqueous environment between pH 3 and pH 8.5.

In order to exclude that this precipitation is due to the denaturation and aggregation of HA in the reaction conditions, HAP was treated, in the presence of carbonate buffer, with DOTA, i.e. the chelating portion of the molecule without of the phenylisothiocyanate portion, at the concentrations used for the reaction.

After 20 h of treatment at 40°C no separation of solid phase (Fig. 2) from the initial solution was evident, this confirms that the insoluble product deriving from the reaction is a chemical entity different from HA.

From Fig. 2 the clear difference between the gelatinous precipitate obtained by the process according to the present invention (right microtube) and the solution deriving from the treatment of HAP with carbonate buffer and DOTA for 20 h at 40°C (left microtube) can be clearly inferred.

Fig. 2A documents the fact that, already in the first hour of reaction of the procedure according to the invention, the formation of a gelatinous precipitate occurs.

Such precipitate, properly purified by repeated washing with deionized water, recovered and freeze-dried, was subjected to IR analysis. The spectrum of such product results to be different from that of p-SCN-Bn-DOTA and from that of HAP.

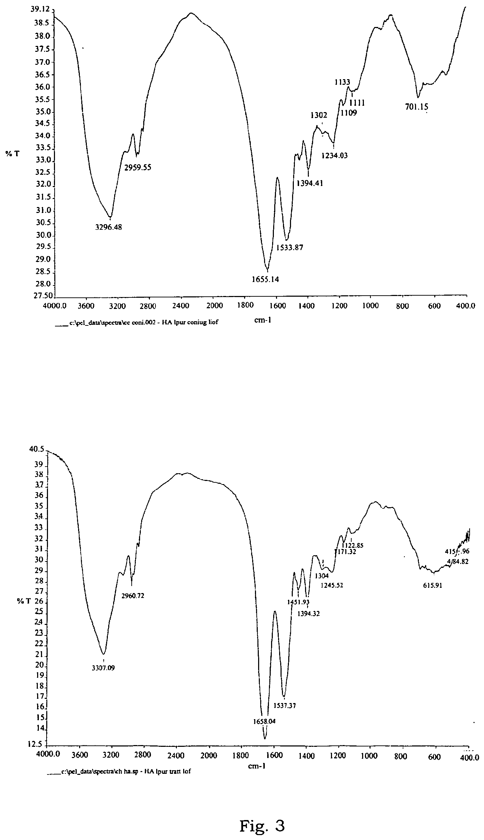

The comparison of the IR spectra in KBr of HAP (Figure 3, bottom) and of HAC (Figure 3, top), shows some spectral differences, attributable to the introduction of the C=S group, characteristic of the presence of a thioureic bond in the HAC conjugate, and described in the work of Ritchie et al. (A spectroscopic study of thiourea derivatives-(III): Infrared and Raman spectra of NN'-disubstituted thioureas Spectrochimica Acta Part A: Molecular Spectroscopy 27, 9, 1971, 1597-1608). Among these, the band enlargement around 1530 cm$^{-1}$ due to the presence of the C=S bond less polar than the C=O bond, the modifications in the spectral region between 1350-1200 cm$^{-1}$, but, mainly the absorbance at 700 cm$^{-1}$ characteristic of the C=S moiety indicating the formation of the thioureic bond responsible of the conjugation of DOTA with HA are noted. These spectral differences are reproducible and significantly relevant, although not particularly emphasized due to the not high degree of substitution of the small molecule of p-SCN-Bn-DOTA (MW 551.6) in the big molecule of HA (MW 66,500).

In Figure 3 a comparison between the IR spectrum of the freeze-dried HAC product object of the invention (top) and of the freeze-dried HAP purified albumin (bottom) subjected to the same reaction conditions, using DOTA instead of p-SCN-Bn-DOTA is shown.

Another evidence for the formation of a chemical entity different from HA is provided by MALDI-TOF mass spectrometry.

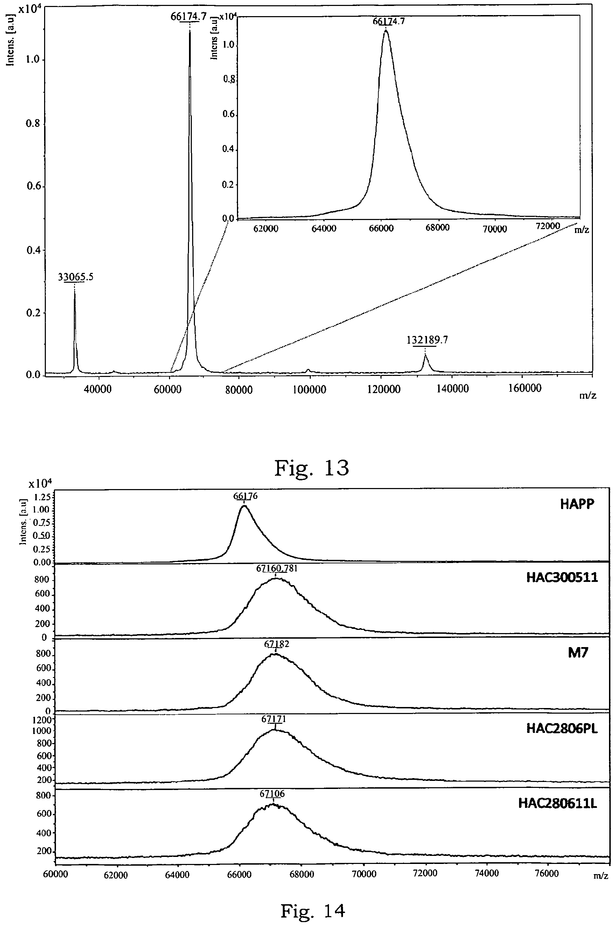

A number of samples of HAC prepared according to the method of the present invention (namely samples M7, HAC300511, HAC280611L and HAC2806PL) were subjected to MALDI-TOF spectrometry, obtaining the spectra of Fig. 14.

All of the HAC samples used for the MALDI-TOF analysis had previously been properly purified by repeated washing with deionized water and used after freeze-drying.

In order to perform the MALDI-TOF analysis, 1 mg of each one of these samples was dissolved in 1 mL H$_2$O, containing 0.1% trifluoroacetic acid (TFA) and the solutions thus obtained were further diluted 1:10 (v/v) with 0.1% TFA.

Several MALDI matrices were tested, using the "dried droplet" MALDI technique, and finally it was found that the best results were obtained with a sinapinic acid matrix and a matrix/analyte ratio of 1:100.

10 mg of sinapinic acid matrix were dissolved in 1 mL of H$_2$O/ACN (50:50, v/v) solution containing 0.1% TFA. The instrumental conditions for the analysis were the following: IS1 = 25kV; IS2 = 23.3 kV; Lens = 6.5 kV; PIE450ns; laser power = 60%.

In Fig. 13 it is reported the MALDI-TOF spectrum of purified HA and in Fig. 14 there are reported the MALDI-TOF spectra of the above-mentioned samples in comparison with the spectrum of HA (HAPP) obtained in the same conditions as for the MALDI-TOF sample preparation described above.

It can clearly be noticed that there is a mass increase of the centroid of the peak of around 1000 Da for each HAC sample, which is attributable to the covalent binding of p-SCN-Bn-DOTA to HA.

The degree of substitution (DS) of HAC can be determined through a spectrophotometric UV methodology that utilizes the decrease of absorbance at 656 nm of the blue-coloured complex, between arsenazo III and Pb(II) due to the concurrent formation of a more stable complex between Pb(II) and DOTA.

In fact, DOTA forms kinetically inert complexes with Pb(II) with a high stability constant ($10^{23}$ -$10^{24}$) at room temperature (Dadachova E. et al. 1999. Spectrophotometric method for determination of bifunctional macrocyclic ligands in macrocyclic ligand-protein conjugates. Nuclear Medicine and Biology 26(8):977-982).

In the following Table 2 spectrophotometric dosing of the content of DOTA macrocycles per mole of HA is reported, expressed as DS ± Cl$_{95}$ or as μmoles DOTA/mg HAC ± Cl$_{95}$, of some samples deriving from three microbatches (A, B, C) of HAC obtained at two different molar ratios of conjugation p-SCN-Bn-DOTA/HAP and related replicates.

Table 2

| Samples/ Replicates | Molar ratio of conjugation p-SCN-Bn-DOTA/HAP | DS nDOTA/nHA (mean ± Cl$_{95\%}$) | μmoles DOTA/mgHAC (mean ± Cl$_{95\%}$) |
|---|---|---|---|
| A/1 | 20/1 | 3.01 ± 0.64 | 0.044 ± 0.009 |
| A/2 | 20/1 | 2.79 ± 0.53 | 0.041 ± 0.008 |
| B/1 | 20/1 | 3.06 ± 0.66 | 0.045 ± 0.009 |
| B/2 | 20/1 | 3.34 ± 0.68 | 0.049 ± 0.010 |
| C/1 | 23/1 | 5.92 ± 0.76 | 0.085 ± 0.010 |
| C/2 | 23/1 | 6.61 ± 0.79 | 0.094 ± 0.011 |
| C/3 | 23/1 | 5.99 ± 0.99 | 0.085 ± 0.013 |

Depending on the reaction conditions used, the conjugated μmoles of p-SCN-Bn-DOTA per mg of HAC generally range from 0.02 (DS=1.3) to 0.2 (DS=15.0), even if the degree of substitution can be raised if needed, properly varying the reaction conditions, till getting near the maximum theoretical degree of substitution of 98, corresponding to reaction on all the nucleophilic sites present on HA: 1 -SH, 16 His, 58 Lys, 23 Arg, in which case the maximum number of conjugated μmoles of p-SCN-Bn-DOTA per mg of HAC is equal to 0.813 and nDOTA/nHA=98.

In particular, samples containing from 0.03 to 0.15 μmoles DOTA/mg HAC, corresponding to a DS ranging between 2 and 10 were studied through complexation with non-radioactive Lutetium ($^{175}$Lu) and with radioisotopes ($^{177}$Lu, $^{111}$In).

The complexation procedure with non-radioactive Lu allows to calculate the yield of incorporation of the metal in the macrocycle conjugated with HA, when this process is carried out at the same temperature (90°C) and time (30 min) conditions used for radioisotopic labelling. These samples were freeze-dried, following purification by ultrafiltration with deionized water from the excess of $LuCl_3$. The ponderal mean yield of the purified and freeze-dried complex HAC-Lu(III), calculated in comparison with the weight of HAC used, is equal to 100.0 $\pm$ 0.3 % (w/w) ($CI_{95}$, n=5).

HAC complexes with radionuclides can be brought to the required degree of purity for diagnostic and therapeutic applications, by using purification techniques known in the art.

By using molar ratios of Lu/HAC between 130/1 and 100/1 for complexation, yields of incorporation of Lu ranging between 40% and 60% are obtained, calculated on the basis of the moles of DOTA introduced per mg of HAC used, determined by a spectrophotometric method. Such yield, calculated using samples that contained not less than 2.4 moles DOTA/moles HA and not more than 4 moles DOTA/moles HA (Example 1) corresponded to a range of incorporation between 20 and 26 nmoles of Lu/ mg HAC (Example 2).

Also this measurement allows to indirectly establish, in addition to the efficiency of complexation in the labelling conditions, the conjugation of p-SCN-Bn-DOTA to HA. Furthermore, it confirms that the conjugation product HAC, although no more soluble in aqueous phase, is able to exert its typical metal complexing action, characteristic of DOTA, also in suspension, i.e. in a heterogeneous phase.

In the same way it was possible to demonstrate that HAC exerts its complexing action towards metals also after freeze-drying and reconstitution of the suspension.

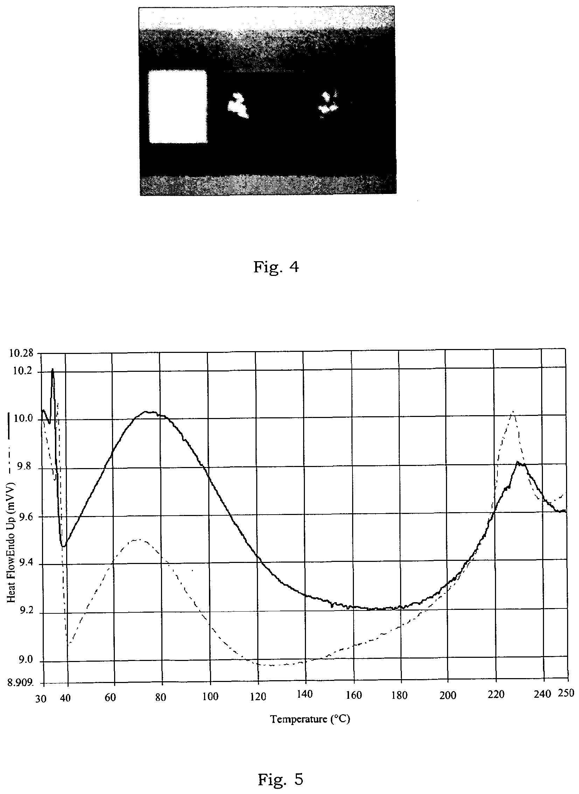

From Figure 4 one can observe that freeze-dried HAC is present in the state of powder (sample indicated with the C letter), while freeze-dried HAP (Fig. 4 on the left) is present with a markedly different aspect of flocky and light solid.

The product can be obtained in sterile form working in conditions of asepsis, starting from medicinal preparations of HA, thus certainly sterilized and apyrogen, and using buffers and reagents wherein sterility is obtained through sterilizing filtration through cellulose membranes with pore sizes $\leq$0.22 $\mu$m. For example, the addition of p-SCN-Bn-DOTA can be performed directly on concentrated or freeze-dried HA as a solution in 0.9% NaCl previously sterilized by filtration. All the other known procedures of sterilization by dry and humid heat in autoclave, by radiations, or other sterilization techniques known in the art can also be used.

Even if with lower yields and purity, the reaction of formation of the insoluble conjugate proceeds also in a sealed vial containing a solution of purified HA and the reagent p-SCN-Bn-DOTA in a suitable molar ratio, during a process of sterilization in autoclave with humid heat, i.e. in conditions of saturated steam.

The thermal behaviour of HAC during labelling is noteworthy. In fact, while HAP tends to coagulate when it is heated for 30 min at 90°C, HAC remains dispersed in suspension.

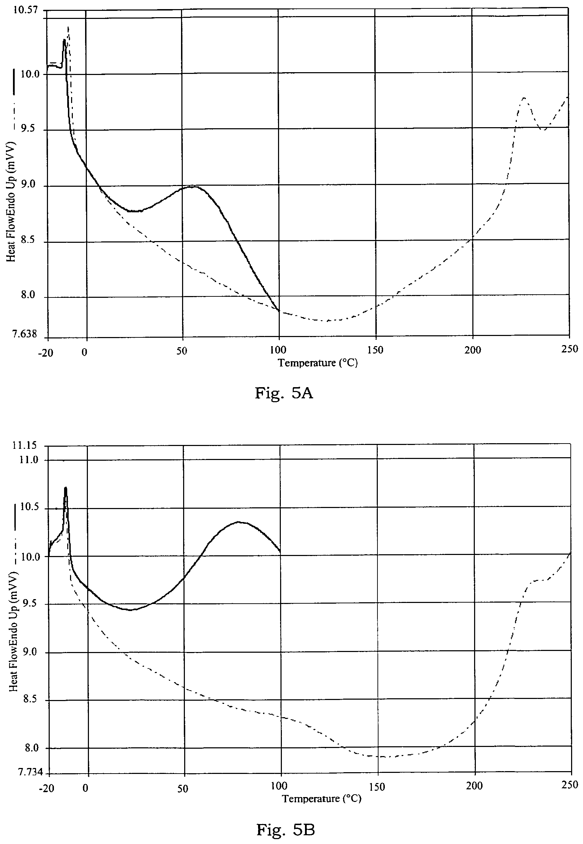

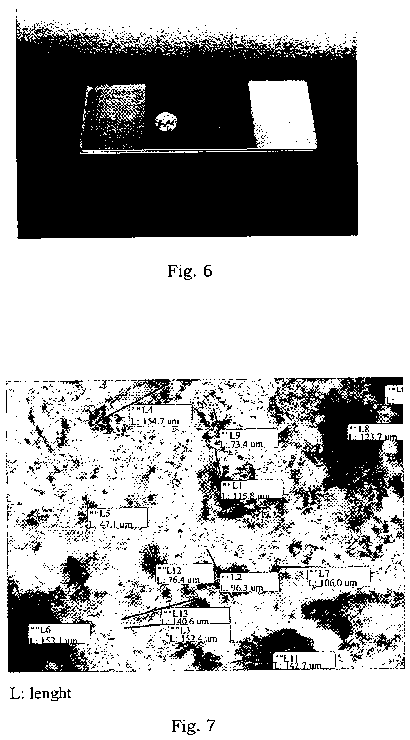

The different thermal behaviour is observed also in the comparison of the DSC profiles (see Fig. 5, 5A, 5B) and in the aspect of the samples after thermal treatment in DSC at 250°C: although both samples changed their colour due to baking, a different behaviour clearly appears: HAP forms a film (Fig. 6 on the left), while HAC remains in a powder state (Fig. 6 on the right).

The size distribution of the particles of conjugate is influenced by the reaction conditions. In mild conditions of temperature and for short times of treatment a conjugate is obtained that, after purification by repeated washing, is present in a suspension of sodium acetate as a colloidal precipitate having mean particle size larger than 10 $\mu$m. In prolonged reaction conditions this size tends to increase, remaining, anyway, smaller than 200 $\mu$m (Fig. 7). Such behaviour is observed also when the precipitate is subjected to different cycles of heating (40°C) and rapid cooling (5-6°C).

The control of the distribution of particle sizes of HAC in suspension can be carried out by centrifugation, in order to remove the particles with mean diameter smaller than 10 $\mu$m and by filtration on filters with controlled porosity in order to remove the particles with mean diameter larger than 100 $\mu$m. The selection of the distribution of particle sizes of HAC, obtained at the state of powder by freeze-drying, is feasible by sieving with certified sieves with minimum diameter of the lumen of the mesh of 20 $\mu$m and maximum diameter of the lumen of the mesh of 100 $\mu$m.

The redispersion of the freeze-dried product can be promoted with the aid of all the additives known to those who are skilled on the art, necessary to improve the stability of the freeze-dried product and its redispersion.

Preparation of a conjugate according to the invention

It is here described, by way of illustration, the preparation of a conjugate according to the present invention.

Materials used and instruments used for said preparation are reported hereinafter.

MATERIALS

Ultrafiltration devices Amicon® Ultra-4 cut-off 10,000 NMWL (Millipore, USA); Dyalisis Cassettes Slide-A-Lyzer® 10K (Pierce, USA); Steril Apyrogenic syringe filters cellulose acetate Albet® Jacs-020-25, (Albet, Spain); 20% Albutein® 50 ml, (Alpha Therapeutic S.p.A, Italy); Human Albumin Baxter - 20 g/100 ml 50 ml bottle (Baxter S.p.A., Italy); Albumin from human, fatty acid free, globulin free, A3782 (Sigma-Aldrich, Germany); DOTA, M-140 (Macrocyclics, USA); p-SCN-Bn-DOTA, B-205 (Macrocyclics, USA); Ammonium acetate p.a. code 1,01116 (Merck, Germany); TraceCERT®, Lead standard for AAS, code 16595 (Sigma-Aldrich, Germany);

Arsenazo III p.a. code 11090 (Sigma-Aldrich, Germany); Sodium Acetate trihydrate p.a. code 1.06267 (Merck, Germany); Sodium Hydrogen Carbonate p.a. code 1.06329 (Merck, Germany); Sodium Chloride 0.9% saline (Eurospital, Italy); Sodium Chloride, p.a. code 1.06404 (Merck, Germany); Sodium Hydroxide solution (1.0N) code 5062-8576 (Agilent Technologies, Germany); Lutetium(III) Chloride code 450960 (Sigma-Aldrich, Germany); Hydrochloric Acid fuming 37% p.a. code 1.00314 (Merck, Germany); Acetic Acid Glacial 100% p.a. code 1.00063 (Merck, Germany); Lutetium ICP/DCP standard solution code 431818 (Sigma Aldrich, Germany), 65% $HNO_3$ trace analysis (Scharlau), ITLC-SG (Varian, USA), $^{177}LuCl_3$ (Perkin Elmer, USA), $^{111}InCl_3$ (Covidien, USA).

INSTRUMENTS

Freezone Lyophilizer 6 liter provided with stoppering tray dryer, LabConco, USA. DSC 7 Perkin Elmer, USA. UV-Vis Spectrophotometer HP 8453, Hewlett-Packard, USA. Orbital Mixing Dry Bath EchoThermTM SC25XT, Torrey-Pines Scientific, USA. Avanti J-30I High-Performance Centrifuge System, Beckman CoulterTM, USA. Microscope alphaphot-2 YS-2 Nikon, Japan provided with Moticam 2300 Motic, China. GC-MS EI HP5890(II)-HP5971A Hewlett-Packard, USA. FT-IR System 2000 Perkin Elmer, USA. The ITLC radiochromatographic profile was recorded by systems of autoradiography imaging with "high performance storage phosphor screen" (Cyclone, Packard BioScience, Meriden, CT, USA). Scintillation analyzer Packard USA. ICP-AES, J.Y. 24, Jobin-Yvon, France, provided with Cetac U-5000AT+ ultrasonic nebulizer, Cetac Technologies, USA. Bench steam sterilizer SteriPlus, De Lama, Italy, MilliQ water (Millipore, USA) or Purelab UHQ (Elga, UK).

Using the above materials and instruments, the preparation was carried out as follows.

0.5 ml of an aqueous solution containing 100 mg of human albumin (HA) are added to an ultrafiltration system with a regenerated cellulose membrane having a cut-off of 10 kDa (NMWL nominal molecular weight), hereinafter called UF10K, following normalization with 3.5 ml of 0.9% NaCl by ultrafiltration at 7400 g for 15 min.

0.9% NaCl is added until a final volume of 3.5 ml is reached and it is gently stirred by oscillation for 5 min in order to guarantee complete mixing of the two phases; ultrafiltration is carried out at 7400 g for 15 min.

The concentrated HA obtained after ultrafiltration, about 0.5 ml, is added with 0.9% NaCl to the final volume of 3.5 ml; after gentle stirring by oscillation for 5 min, in order to guarantee complete mixing of the two phases, centrifugation at 7400 g for 15 min is carried out. Such operation is repeated for at least other 3 times or until complete disappearance of the absorbance of N-acetyl triptophan in the ultrafiltrate, measured at 280 nm.

Purified human albumin (HAP) is resuspended with about 1.5 ml of 0.9% NaCl to obtain a 2 ml stock solution of HAP containing about 50 mg/ml of HA, which concentration is carefully determined by UV dosing at 280 nm versus a solution of pure standard HA, prepared dissolving 5.00 mg carefully weighted of pure standard HA free from fatty acids and globulins in 10 ml of 0.9% NaCl. The HAP stock solution is stored in the fridge or can be freeze-dried as such.

A 1.5 ml polypropylene tube is filled with 0.5 ml of HAP stock solution (or 25 mg of freeze-dried HAP reconstituted with deionized water) and there are added 5.00 to 6.00 mg of p-SCN-Bn-DOTA that are solubilised by stirring and possibly by sonication. A 60 $\mu$l aliquot of carbonate buffer at pH 9.0 in 0.9% NaCl, with a concentration ranging between 0.1M and 0.5M depending on the desired DS (see Fig. 1), can be added to such solution.

The tube with the reaction mixture is subjected to heating at 40°C and orbital shaking at 300 rpm for 1 h on a thermoshaker. After such time a very viscous white gelatinous precipitate is formed, which marks the beginning of the conjugation reaction, at this point additional 250 $\mu$l of 0.9% NaCl are added and it is vortexed for 1 min. The sample is subjected to heating at 40°C and orbital shaking at 300 rpm for additional 15 min on a thermoshaker. At this point, additional 250 $\mu$l of 0.9% NaCl are added, it is vortexed for 1 min and then heating at 40°C under orbital shaking at 300 rpm on a thermoshaker is continued for 16 hours.

The raw product of the reaction, i.e. the suspension of conjugated albumin HA-p-SCN-Bn-DOTA (HAC) in the reaction medium, is transferred with the aid of 0.9% NaCl in an ultrafiltration device having a cut-off of 10 kDa; the volume of the suspension is brought to 3.5 ml with 0.9% NaCl and centrifugation at 7400 g for 15 min is carried out, following 5 min of gentle stirring by oscillation; such washing is repeated other two times bringing the volume of the concentrated retained by the ultrafiltration device to the volume of 3.5 ml with 0.9% NaCl. Then washing is performed always on the same ultrafiltration device with 1M sodium acetate buffer at pH 5, bringing the volume of the suspension to 3.5 ml and ultrafiltering by centrifugation at 7400 g for 15 min. The latter washing is repeated for at least 3 times or until complete disappearance of the UV absorbance spectrum of the macrocyclic reagent in the ultrafiltrate. The concentrated product, about 0.5 ml of suspension containing HAC in 1M sodium acetate buffer at pH 5, is transferred with the aid of the same buffer into a polypropylene tube of suitable capacity and brought to the final volume of 1.5 ml. Such suspension is stored in the fridge or freeze-dried.

SPECTROPHOTOMETRIC DETERMINATION OF THE DEGREE OF CONJUGATION OF p-SCN-Bn-DOTA WITH HA

A stock solution of Pb(II)-AA(III) (AA = arsenazo) in 0.15M AcONH$_4$, pH=7.00 is prepared, containing 67.62 $\mu$mol/l of Pb(II) and 140 $\mu$mol/l of AA(III). Such stock solution must be stored protected from light, at a temperature of 2-6°C and used at room temperature within one day after its preparation. A stock solution of DOTA 0.344mM in AcONH$_4$ (0.15M, pH=7.00) is prepared.

For the calibration curve, six standard solutions are prepared, each equal to a volume of 4.2 ml, containing increasing concentrations of DOTA from 0 to 0.041mM.

3.4 ml of Pb(II)-AA(III) stock solution, 200 $\mu$l of 1M NaCl (in ammonium acetate) and a variable volume of DOTA stock solution (0-500 $\mu$l), depending on the desired final concentration, are added in each solution. The final volume of 4.2 ml for each standard solution is reached adding a suitable volume of AcONH$_4$ (0.15M, pH=7.00). For each solution, absorbance at 656 nm is determined, 10 min after its preparation at room temperature and protected from light. The reading is corrected subtracting the absorbance of a solution comprised of 4.0 ml of AcONH$_4$ buffer (0.15M, pH=7.00) and 200 $\mu$l of 1M NaCl.

The sample solution is prepared adding 0.90 to 1.10 mg of HAC into a solution containing 3.4 ml of Pb(II)-AA(III) stock solution, 200 $\mu$l of 1M NaCl, 600 $\mu$l of AcONH$_4$ buffer (0.15M, pH=7.00). Then absorbance at 656 nm is determined, 10 min after its preparation, at room temperature and protected from light. Results are expressed as $\mu$moles of DOTA/mg HAC or as DS = n moles DOTA/n moles HA.

DS is calculated through the following equation:

$$DS = \frac{MW_{HA}}{\dfrac{\mu gHAC}{\mu moles\ DOTA} - MW_{DOTANCS}}$$

where

$MW_{HA}$ = molecular weight of HA

$\mu$gHAC = weighted micrograms of HAC freeze-dried sample

**$\mu$moles DOTA** = micromoles of HA-conjugated DOTA calculated from the calibration curve

$MW_{DOTANCS}$ = molecular weight of p-SCN-Bn-DOTA

DIFFERENTIAL SCANNING CALORIMETRY (DSC)

Carefully weighted samples about 2.5 $\pm$ 0.5 mg were subjected to a thermal gradient of 10°C/min under a flow of nitrogen in an open aluminium melting-pot. Two types of scannings were carried out, from 30°C to 250°C and scannings from -20°C to 250°C with pre-heating from -20°C to 100°C.

FREEZE-DRYING PROCEDURE

The HAC sample, placed in a proper polypropylene tube, is frozen in a lyophilizer at - 32°C. When thermal equilibrium is reached, as measured by a thermal probe immersed in the sample, a cold trap at -50°C is actuated and freeze-drying is started by generating vacuum. The process of freeze-drying, monitored by the thermal probe, for volumes of HAC suspension ranging from 1 to 10 ml, lasts between 16 to 24 hours.

When the temperature of the product equals the temperature of the plate, the sample is subjected to secondary drying, at 30°C under vacuum, until reaching a constant weight of the freeze-dried product. The freeze-dried product is properly stored between 2 and 6°C protected from humidity.

Such procedure is also adopted to freeze-dry the HAP solution in 0.9% NaCl and to obtain a freeze-dried product to reconstitute to the original volume of suspension with sterile water, or to freeze-dry HAP samples following purification from NaCl by dialysis or ultrafiltration.

COMPLEXATION OF HAC WITH $^{175}$Lu, NON-RADIOACTIVE ISOTOPE.

8.00 mg of carefully weighted HAC are dispersed into 400 $\mu$l of 1M sodium acetate at pH 5 in a polypropylene tube. 100 $\mu$l of a solution containing 4.0 mg of LuCl$_3$ dissolved in HCl 0.05N are added to the dispersion. The tube is placed in a thermoshaker and the reaction is carried out at 90°C for 30 min under stirring at 400 rpm.

The cooled suspension is transferred into an ultrafiltration device and washed three times with 3 ml of 0.9% NaCl then with 3 ml of deionized water; such washings are sufficient to eliminate the excess of LuCl$_3$, given the absence of Lu, or its presence below of the limit of detection of the ICP-AES method, in the ultrafiltrate of the last washing. The aqueous suspension is then freeze-dried according to the procedure described above.

ICP-AES DETERMINATION OF $^{175}$Lu COMPLEXED WITH HAC (HAC-$^{175}$Lu)

All the determinations were performed at the wavelength of 261.542 nm by a calibration curve built with 6 aqueous solutions of increasing concentration containing up to 3 $\mu$g/ml of standard Lu.

The amount of $^{175}$Lu is determined in a carefully weighted HAC-Lu sample, in the range 0.90 to 1.10 mg. Such sample is dissolved in a polypropylene tube with 1 ml of 65% HNO$_3$ and kept at room temperature for 1 hour, before analysis the sample is diluted with 1 ml of UHQ water. The mean recovery of Lu in artificial samples (n=5) containing 1.0 mg of HAP is equal to 99.4 $\pm$ 0.2 % of $^{175}$Lu added therein.

HAC RADIOLABELLING

Radiolabelling is carried out with both $^{111}$In and $^{177}$Lu at the specific activity of 1 mCi/mg, using HAC (10-20 mg/ml) suspended in 1.0M sodium acetate pH 5.0.

A suitable volume of $^{177}$LuCl$_3$ or $^{111}$InCl$_3$ in 0.05N HCl is dosed in a sterile polypropylene tube already containing the HAC suspension in sodium acetate. After mixing it is heated at 90°C for 30 min.

RADIOCHEMICAL PURITY (RCP)

Radiochemical purity (RCP) is evaluated in triplicate for each radiolabelled sample by instant thin-layer chromatography (ITLC).

One aliquot of the radiolabelled suspension is mixed with 0.1 ml of a 2.5mM solution of diethylene-triamine-pentaacetic acid (DTPA) at pH 5.0. 5 $\mu$l of the radioactive suspension are deposed on an ITLC SG support and the development by ascending chromatography with 0.9% NaCl as mobile phase is carried out. In this chromatographic system, the insoluble HAC-radionuclide complex does not migrate, while the possibly present free radionuclide, bound to DTPA, migrates along with the solvent front. The radiochromatographic profile is determined by an autoradiographic system that uses a high performance storage phosphor screen (Cyclone, Packard BioScience, Meriden, CT, USA) and the radiochemical purity (RCP) is consequently calculated.

PROCEDURE FOR THE EVALUATION OF MICROBIOLOGICAL CONTAMINATION

The evaluation of microbic contamination, is carried out according to the guidelines for fill-tests, in an internal microbiology laboratory. Each vial containing 1.5 ml of HAC suspension in 1.0M sodium acetate at pH 5.0 is incubated in a thermostated oven (Heraeus Instruments series 6000) at (37 $\pm$ 1)°C for 14 days, and then qualitatively analyzed for the presence of any turbidity. In the case of a positive sample, 1 ml of this is plated on a Petri capsule to quantitatively evaluate the microbiological growth of the sample.

ITLC CHROMATOGRAPHY

The chromatographic mobility of HAC on a ITLC SG support identical to that used for the RCP evaluation, in comparison with HA and purified HA (HAP) is assessed as follows.

5 $\mu$l of a suspension containing 8 mg HAC in 1 mL of 1M sodium acetate with pH = 5, or in 0.9% NaCl or in water are deposed on the ITLC SG support and the development by ascending chromatography with 0.9% NaCl as mobile phase is carried out.

The plates, after chromatographic development and drying under ambient conditions for 1 hour, are kept in a drier containing silica gel and then subjected to UV-VIS scanning in a TLC Scanner III Camag (CH). The reflectance at 280 nm is measured.

The same procedure is carried out with HA and HAP, using respective solutions containing 25 mg/mL.

Example 1

SPECTROPHOTOMETRIC DETERMINATION OF THE DEGREE OF CONJUGATION OF p-SCN-Bn-DOTA WITH HA ON TWO HAC SAMPLES (BATCH 041010)

The procedure described in the previous section entitled "SPECTROPHOTOMETRIC DETERMINATION OF THE DEGREE OF CONJUGATION OF p-SCN-Bn-DOTA WITH HA" was carried out starting from a DOTA stock solution = 0.0178 mg/ml

$R^2$= 0.9932 $S_{Yx}$= 0.0175

Table 3

| Abs (Au) | mmoles of DOTA |
|---|---|
| 1.69 | 0.000 |
| 1.63 | 0.035 |
| 1.53 | 0.069 |
| 1.42 | 0.104 |
| 1.30 | 0.139 |
| 1.21 | 0.174 |

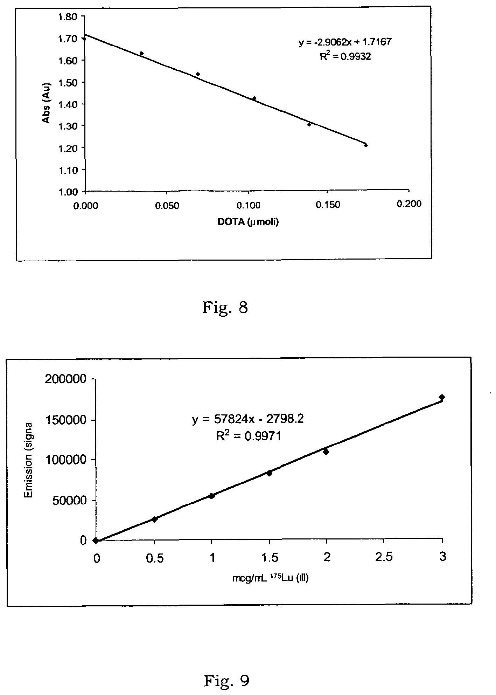

Based on the data reported in Table 3 the calibration curve of Figure 8 was built.

By virtue of the obtained calibration curve the values of degree of conjugation (DS) reported in the following Table 4 were calculated for the two samples.

Table 4

| Sample | HAC Batch | Absorbance (Au) | HAC (mg) | $\mu$moles of DOTA/mg HAC | DS (nDOTA/nHA) (95% confidence interval) |
|---|---|---|---|---|---|
| 1 | 041010 | 1.582 | 1.02 | 0.0454 | 2.40-3.72 |
| 2 | 041010 | 1.576 | 0.98 | 0.0493 | 2.66-4.02 |

Example 2

The procedure described in the previous section entitled "ICP-AES DETERMINATION OF $^{175}$Lu COMPLEXED WITH HAC (HAC-$^{175}$Lu)" was carried out on two HAC-$^{175}$Lu samples.

As explained in said section, first the calibration curve of Figure 9 was built, with six aqueous solutions of increasing concentration, containing up to 3 $\mu$g/ml of standard $^{175}$Lu, based on the values reported in the following Table 5.

$R^2$ = 0.9971 $S_{Yx}$= 3776.93

Table 5

| $\mu$g/ ml | Emission at 261.542 nm |
|---|---|
| 0 | 240 |
| 0.5 | 26123 |
| 1 | 53890 |
| 1.5 | 82767 |
| 2 | 107743 |
| 3 | 175043 |

By virtue of the obtained calibration curve, the values of the amounts (nmoles/mg of HAC-Lu) of complexed $^{175}$Lu reported in the following Table 6 were calculated.

Table 6

| Sample | HAC Batch | HAC-Lu | Emission (sample-matrix) | HAC-Lu (mg) | (nmoles Lu/mg HAC-Lu) (95% confidence interval) |
|---|---|---|---|---|---|
| 1 | 041010 | 051010 | 126203 | 1.16 | 20.8-23.3 |
| 2 | 041010 | 051010 | 118886 | 1 | 22.8-25.5 |

Example 3

The procedure described in the previous section entitled "RADIOCHEMICAL PURITY (RCP)" was carried out on a HAC sample (batch 110610) labelled with [111]In.

Sample Y (Batch 110610): 16.7 mg/ml HAC in 1M sodium acetate pH 5.

Specific activity of HAC labelled with $^{111}$In: 1 mCi/mg.

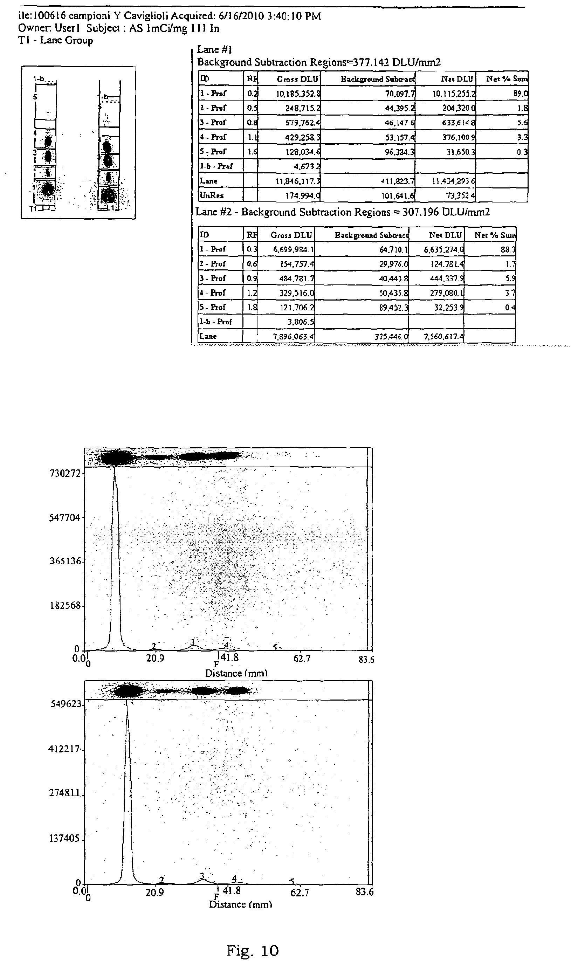

Results described in Figure 10 were obtained.

Example 4

The procedure described in the previous section entitled "RADIOCHEMICAL PURITY (RCP)" was carried out on a HAC sample (batch 110610) labelled with [177]Lu.

Sample Y (Batch 110610): 16.7 mg/ml HAC in 1M sodium acetate pH 5.

Specific activity of HAC labelled with $^{177}$Lu: 1 mCi/mg

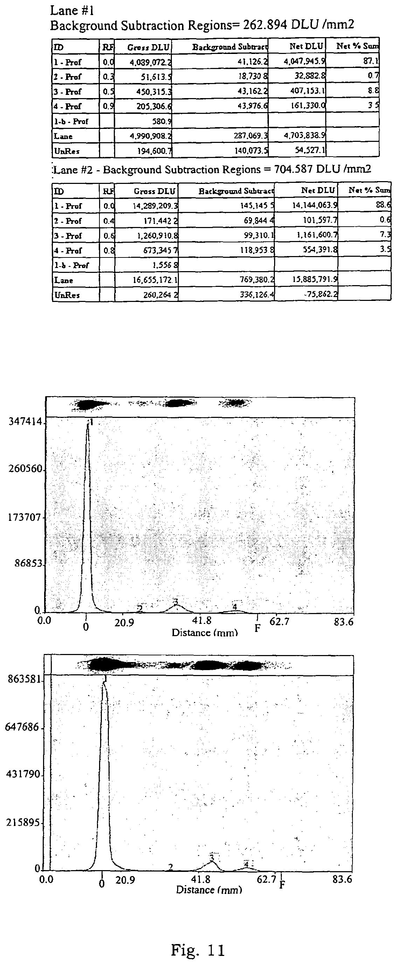

Results described in Figure 11 were obtained.

Example 5

The procedure described in the previous section entitled "RADIOCHEMICAL PURITY (RCP)" was carried out on a HAC sample (batch 201109) labelled with [177]Lu.

Sample C (201109): 25 mg/ml HAC in 1M sodium acetate pH 5

Specific activity: $^{177}$Lu 10.2 Ci/mg

Specific activity of HAC labelled with $^{177}$Lu: 2 mCi/mg

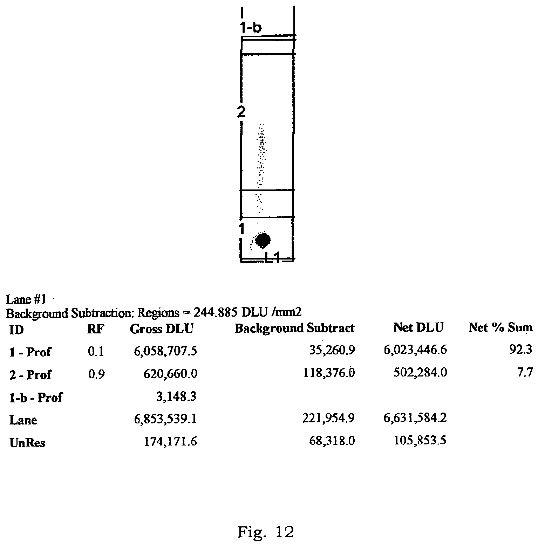

Results described in Figure 12 were obtained.

Example 6

The stability of HAC labelled with $^{177}$Lu in saline, was evaluated measuring its RCP 6, 24, 48 and 144 hours after the labelling of HAC with [177]LuCl$_3$. Hereinafter all the experimental conditions of the stability test are reported and the values of %RCP are reported in the following Table 7.

HAC batch: Y110610

Date: 2010-06-30 Time: 10:00

Labelling

$^{177}LuCl_3$

Preparation date 2010-06-22 Time 18:00

Specific activity $^{177}LuCl_3$: 20.37 Ci/mg

Volume:600 μl Concentration: 0.016 mCi/μl at calibration

Activity calculated at date 2010-06-30 and time 18:00 of calibration: 10 mCi

Activity calculated at date 2010-06-30 and time 10:00 of preparation: 10.2 mCi

Volume of suspension of HAC = 120 μl (corresponding to 2000 μg HAC)

Specific activity of labelling: 1 mCi/mg

Incubation: 90°C for 30 min

Final volume: 240 μl Measured activity: 2 mCi (RCP control)

The residual volume from preparation by dilution of the samples of Example 7, was stored for 6 days and the RCP monitored according to the time intervals reported in the table. After 6 days, the sample showed the same RCP.

Table 7

| t (hours) | %RCP |
| --- | --- |
| 0 | 96 |
| 6 | 96 |
| 24 | 96 |
| 48 | 96 |
| 144 | 96 |

Example 7

STABILITY OF HAC LABELLED WITH $^{177}Lu$ IN TISSUES

Two histopathological samples of spheroidal form, weight and diameter as reported in Table 8,

Table 8

| | Weight (mg) | Diameter (mm) |
| --- | --- | --- |
| Sample A | 50.5 | 4.5 |
| Sample B | 73.6 | 5.1 |

derived from a surgical piece of mammalian carcinoma, containing internal tumoral proliferations, were inoculated with two doses of HAC-$^{177}Lu$, prepared as follows.

HAC batch: Y110610

Date: 2010-06-30 Time: 10:00

Labelling

$^{177}LuCl_3$

Preparation date 2010-06-22 Time 18:00 Specific activity $^{177}LuCl_3$: 20.37 Ci/mg

Volume: 600 $\mu$l Concentration: 0.016 mCi/$\mu$l at calibration

Activity calculated at date 2010-06-30 and time 18:00 of calibration: 10 mCi

Activity calculated at date 2010-06-30 and time 10:00 of preparation: 10.2 mCi

Volume of suspension of HAC = 120 $\mu$l (corresponding to 2000 $\mu$g HAC)

Specific activity of labelling: 1 mCi/mg

Incubation: 90°C for 30 min

Final volume: 240 $\mu$l Measured activity: 2 mCi (RCP control)

Diluted 20 times

2 Doses of 100 pCi/240 $\mu$l prepared

Said samples inoculated with HAC-$^{177}$Lu were stored in 5 ml of PBS (sterile phosphate buffered saline without $Ca^{++}$ and $Mg^{++}$) for 24 hours at 37°C.

After 24 hours the radioactivity of the two samples, measured by gamma counting, remained unchanged in comparison to the reading at the time of inoculation, while no radioactivity was detected in the storage solutions without inoculated samples.

Such samples stored at 2-6°C in PBS for additional 120 h showed to be as radioactive as at the time of inoculation while the presence of radioactivity was not detected in the storage solutions without inoculated samples for all the duration of the experiment.

Example 8

The procedure described in the previous section entitled "ITLC CHROMATOGRAPHY" was carried out to assess the chromatographic mobility of HAC on a ITLC SG support, in comparison with HA.

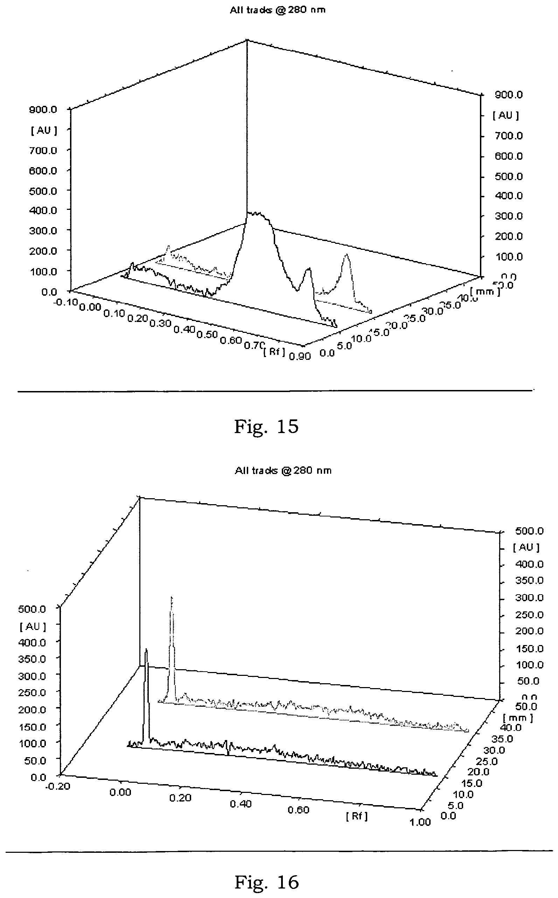

Figure 15 is ITLC chromatogram of commercial HA and purified HA (HAP). In particular, as the HA the commercial product Grifols diluted to 25 mg/mL was used, and HAP was obtained from said HA, according to the previously described purification procedure by ultrafiltration.

The chromatogram of HA is the dark gray-colored one and the chromatogram of HAP is the light gray-colored one.

Two bands are visible in the HA chromatogram at $R_f$ = 0.75 and 0.89, while only one band with $R_f$ = 0.89 is visible in the HAP chromatogram.

Figure 16 is a ITLC chromatogram of an HAC sample according to the invention. The dark gray-colored chromatogram was obtained by deposing 5 $\mu$l of a suspension containing 8 mg HAC in 1 mL of 1M sodium acetate with pH = 5 on the ITLC SG support and then following the procedure described under previous section "ITLC CHROMATOGRAPHY".

The light gray-colored chromatogram was obtained by deposing 5 $\mu$l of a suspension containing 8 mg HAC in 1 mL of 1M sodium acetate with pH = 5 on the ITLC SG support, after heating said suspension for 30 minutes at 90°C, and then following the procedure described under previous section "ITLC CHROMATOGRAPHY".

The step of heating the suspension at 90°C for 30 minutes, before deposing it, is aimed at simulating the conditions of a radiolabeling step.

It can be noticed from both chromatograms of Fig. 16 that HAC does not migrate with a 0.9% NaCl mobile phase, whereas HA and HAP migrate in these conditions. It has also been found that HAC does not migrate either when the mobile phase is water. This is clear evidence that HAC is not soluble in water.

From Figure 16 it is also apparent that a thermal treatment simulating the radiolabeling conditions does not change the chromatographic behaviour of HAC.

Example 9

The procedure described in the previous section entitled "RADIOCHEMICAL PURITY (RCP)" was carried out on a HAC sample (batch HAC081111) labelled with [111]In.

Sample Y (Batch HAC081111SA): 16.7 mg/ml HAC in 1M sodium acetate pH 5.

Specific activity of HAC labelled with $^{111}$In: 1 mCi/mg.

1 mL of a solution of 1:1 v/v human plasma/ultrapure water was added to 1 mL of the above sample, followed by incubation at 37°C in a thermostated bath.
Radiochemical purity (RCP) was assessed after 24 h, 48 h and 144 h.
An optimal stability of HAC in plasma was found after all of the above measurements.

Example 10

A series of solubility assessments on HAC were carried out at temperatures of 37°C and 25°C.
HAC prepared according to the procedure previously outlined, after being properly purified by repeated washing with deionized water, recovered and freeze-dried, was subjected to the following solubility test.
4 mg of such purified HAC were suspended in 1 mL of an appropriate buffer within a 1.5 mL microcone and kept on an orbital mixing heating dry bath Torrey Pines Scientific Inc. SC 25, at 500 rpm for 1 hour, followed by centrifugation on a Beckmann ultracentrifuge at 27000 g for 20 min.
The supernatant was separated and transferred into a 800 $\mu$l microcuvette to detect the presence or the absence of a UV absorbance, in particular at 280 nm.
The underlying pellet, after at least 4 washings with 1 mL water and final centrifugation to remove the washing water, was lyophilized and weighed.
The above procedure was carried out at both 25°C and 37°C, using as buffers a solution of 0.01M acetic acid/sodium acetate with pH = 4.0 and a 0.01 M phosphate buffer with pH = 8.0.
In all cases, no UV absorbance was detected for the supernatant and the final weight of each sample was substantially unchanged.

Example 11

$^{64}$Cu radiolabeling of HAC and $\mu$PET analysis

HAC was labeled with the positron emitting radiosiotope [64Cu]-Copper ($t_{1/2}$ = 12.7 h and decay properties $\beta$- 38.4%, $\beta^+$ 17.8%) in order to evaluate through positron emission tomography (PET) studies its capability to be retained by mammary glands.
$^{64}$CuCl$_2$ was prepared according to the method disclosed in Matarrese et al. Applied Radiation and Isotopes, 2010, (68), 5-13 and used to label HAC according to the procedure under previous section "HAC RADIOLABELLING".
The radiochemical purity of the labeled HAC was verified by the ITLC procedure that has already been herein described. The radiolabeling yield was 100% and the specific activity of the final product was 0.9 mCi/mg.
The following test was carried out with the $^{64}$Cu-labeled HAC.
Two adult rats, female, were housed in a controlled environment with a 12-hours light/dark cycle and maintained on Mucedola Certified Rodent Diet (4RF21 GLP Certificate) and water *ad libitum.*
Animals were anesthetized with a gaseous mixture of oxygen and isoflurane and they were injected in the upper mammary glands, in particular the thoracic left and right mammary glands, with a suspension of $^{64}$Cu-HAC in 1M acetate buffer. ECG and respiration were constantly monitored during whole analysis. In the following table were reported analysis data of the two animals.

| ID rat | Weight | Net Injected Activity | | Injected Compound | | Injection time | |
|---|---|---|---|---|---|---|---|
| | | Left gland | Right gland | Left gland | Right gland | Left gland | Right gland |
| 1 | 244 g | 220 $\mu$Ci | 294 $\mu$Ci | 30$\mu$L / 245$\mu$g | 40 $\mu$L / 326$\mu$g | 23.19 13/12/2011 | 23. 15 13/12/2011 |
| 2 | 235 g | 147 $\mu$Ci | 220 $\mu$Ci | 20 $\mu$L / 163$\mu$g | 30 $\mu$L/ 245$\mu$g | 00.32 14/12/2011 | 00.28 14/12/2011 |

A $\mu$-PET imaging scan was carried out on the whole body for 30 minutes at several times after the radiotracer injection (T = 0), namely at the following times:
The PET scans were reconstructed using the ordered subset expectation maximization (OSEM) algorithm.

A $\mu$-CT imaging scan was also carried out; the $\mu$-CT projection images were acquired over a rotation of about 200° at 80 kVp, 450 Ma and a resolution of 90 $\mu$m. The $\mu$-CT

| ID Animal | Scan Time (Hours) |
|-----------|-------------------|
| 1 | T=0; T= 2.5; T=1 9.66; T=43 |
| 2 | T=0; T=2.33; T=19.5; T=38.25 |

images were reconstructed to give a volume data set.

After the data set reconstruction, PET and CT images were overlapped to analyze and quantify the rate of $^{64}$Cu-HAC in the mammary glands. Quantification was performed by drawing regions of interest (ROIs) of the target sites.

PET imaging demonstrated that the tracer behaviour was substantially the same in the two animals, with the tracer being distributed, after injection, substantially only in the mammary glands.

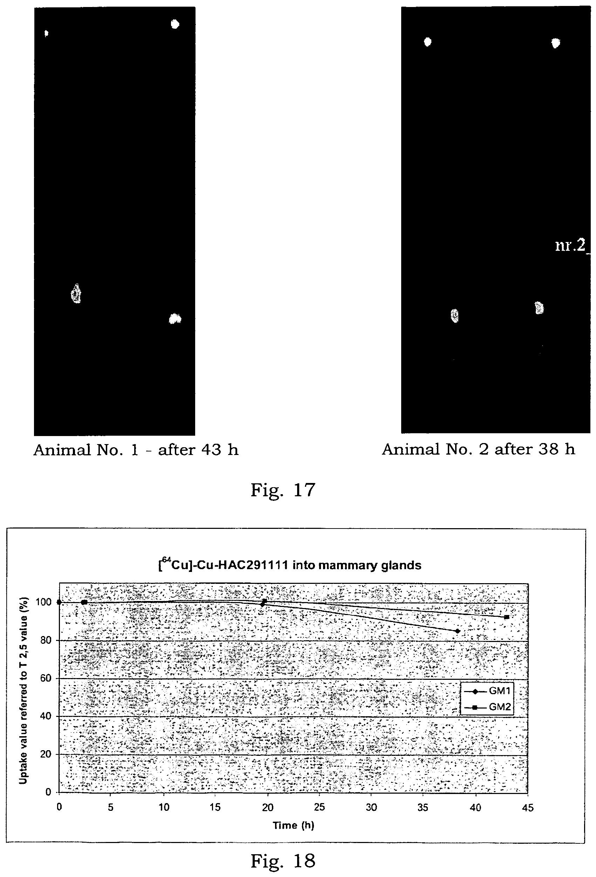

In Fig. 17 there are reported two images obtained by overlapping $\mu$-PET images (that give information on radiotracer distribution) and $\mu$-CTimages (that give morphological information to localize the radioactivity) taken from animal No. 1 and No. 2, 43 h and 38 h after the injection, respectively. In the upper part of the images, near the hind legs, are located two reference points, consituted by a high-density object imbibed with $^{64}$CuCl$_2$ , the radioisotope used as reference in both PET and CT images to allow their overlapping.

It can be noticed that after about 40 hours all the radioactivity is located in the mammary glands.

In Fig. 18 there is illustrated the relative uptake value of the labeled HAC referred to the value measured after 2.5 hours upon injection.

From this figure it is apparent that the labeled HAC is retained after injection in the mammary glands of both animals at least until 40 hours.

## Claims

1. A process for preparing a conjugate of human albumin (HA) and 2-(4-isothiocyanatobenzyl) -1,4,7, 10-tetraazacyclododecane-1,4,7, 10-tetraacetic acid (p-SCN-Bn-DOTA), comprising the steps of:

   a) reacting HA with p-SCN-Bn-DOTA in an aqueous medium at a temperature between 35°C and 45°C, thus forming a suspension, containing a solid phase suspended in a liquid phase;
   b) separating said solid phase, containing said conjugate, from said liquid phase, possibly containing unreacted p-SCN-Bn-DOTA.

2. The process according to claim 1, wherein said separation step b) consists of a filtration or ultrafiltration step.

3. The process according to claim 2, wherein a solid obtained from said filtration step or, respectively, a retentate obtained from said ultrafiltration step is subjected to freeze-drying.

4. The process according to any one of claims 1 to 3, wherein said reaction between HA and p-SCN-Bn-DOTA is carried out at a temperature of 38-42°C for 4-24 h, preferably 8-21 h.

5. The process according to any one of claims 1 to 4, wherein said aqueous medium is an aqueous solution with a ionic strength of 0.1-0.5M, in particular a solution of NaCl 0.9%, optionally buffered at pH 4-8.5.

6. The process according to any one of claims 2 to 5, wherein a cut-off of 10 kDa is used in said ultrafiltration step.

7. The process according to any one of claims 1 to 6, wherein the molar ratio between p-SCN-Bn-DOTA and HA is between 100:1 and 1:1, preferably between 30:1 and 16:1.

8. The process according to any one of claims 1 to 7, wherein the HA that is reacted with p-SCN-Bn-DOTA in said step a) has previously been purified by ultrafiltration or dialysis with a membrane having a cut-off of 10 kDa, in the presence of a solution with a ionic strength of 0.1-0.5M, in order to remove thermal stabilizers that are present in the commercially available HA, in particular sodium caprylate and sodium acetyl tryptophanate.

9. A conjugate of human albumin (HA) and 2-(4-isothiocyanatobenzyl)-1,4,7,10-tetraazacyclododecane-1,4,7,10-tetraacetic acid (p-SCN-Bn-DOTA), which is insoluble in aqueous medium at a pH of 3-8.5 and whose IR spectrum shows an absorption at about 700 cm$^{-1}$, obtainable by the process according to any one of claims 1 to 8.

10. A complex consisting of a conjugate of HA and p-SCN-Bn-DOTA (HAC) according to claim 9 and a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu.

11. The complex according to claim 10, wherein said radioactive isotope is selected among the group consisting of $^{111}$In, $^{177}$Lu, $^{90}$Y, $^{67}$Cu, $^{64}$Cu and radioactive lanthanides and is preferably $^{177}$Lu.

12. The complex according to any one of claims 10 and 11 for therapeutic use.

13. The complex according to any one of claims 10 and 11 for use in the *in vivo* localization of radionuclides in organs and tissues of the human body for diagnostic or therapeutic purpose.

14. The complex according to any one of claims 10 and 11 for use in the localization of mammalian neoplastic lesions by the R.O.L.L. ("Radioguided Occult Lesion Localization") methodology.

15. A process for preparing an HAC complex according to any one of claims 10 and 11, comprising the steps of:

   - dispersing a conjugate according to claim 9 in an aqueous buffer at pH 4-6;
   - adding a water-soluble compound containing an ion of a radioactive isotope of an element selected among the group consisting of Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe and Cu, and
   - heating under stirring at a temperature of 30-90°C for 20-40 minutes.

16. The process according to claim 15, wherein said water-soluble compound is $^{177}$LuCl$_3$.

17. The process according to any one of claims 15 or 16, wherein said aqueous buffer is an acetic acid/acetate buffer, preferably 1M with a pH of 5.

**Patentansprüche**

1. Verfahren zum Zubereiten eines Konjugats von humanem Albumin (HA) und 2-(4-isothiocyanatobenzyl)-1,4,7,10-tetraazacyclododecan-1,4,7,10-Tetraessigsäure (p-SCN-Bn-DOTA), umfassend die Schritte:

   a) Reagieren von HA mit p-SCN-Bn-DOTA in einem wässrigen Medium bei einer Temperatur zwischen 35°C und 45°C, wobei eine Suspension gebildet wird, die eine feste Phase enthält, welche in einer flüssigen Phase suspendiert ist;
   b) Trennen dieser festen Phase, die das Konjugat enthält, von der flüssigen Phase, die möglicherweise unreagiertes p-SCN-Bn-DOTA enthält.

2. Verfahren gemäß Anspruch 1, wobei der Trennungsschritt b) in einem Filtrations- oder Ultrafiltrationsschritt besteht.

3. Verfahren gemäß Anspruch 2, wobei ein aus dem Filtrationsschritt erhaltener Feststoff oder ein aus dem Ultrafiltrationsschritt erhaltenes Retentat einer Gefriertrocknung unterzogen wird.

4. Verfahren gemäß einem der Ansprüche 1 bis 3, wobei die Reaktion zwischen HA und p-SCN-Bn-DOTA bei einer Temperatur von 38-42°C während 4-24 h, vorzugsweise 8-21 h, durchgeführt wird.

5. Verfahren gemäß einem der Ansprüche 1 bis 4, wobei das wässrige Medium eine wässrige Lösung mit einer Ionenstärke von 0,1-0,5 M, insbesondere eine 0,9%ige NaCl-Lösung, optional bei pH 4-8,5 gepuffert, ist.

6. Verfahren gemäß einem der Ansprüche 2 bis 5, wobei eine Obergrenze von 10 kDa in dem Ultrafiltrationsschritt verwendet wird.

7. Verfahren gemäß einem der Ansprüche 1 bis 6, wobei das Molverhältnis zwischen p-SCN-Bn-DOTA und HA zwischen 100:1 und 1:1 ist, vorzugsweise zwischen 30:1 und 16:1.

8. Verfahren gemäß einem der Ansprüche 1 bis 7, wobei das HA, welches in Schritt a) mit p-SCN-Bn-DOTA reagiert, vorher durch Ultrafiltration oder Dialyse durch eine Membran mit einer Obergrenze von 10 kDa in Anwesenheit einer Lösung mit einer Ionenstärke von 0,1-0,5 M, insbesondere Natriumcaprylat und Natriumacetyltryptophanat, gereinigt worden ist, um thermische Stabilisatoren zu entfernen, die in dem kommerziell erhältlichen HA vorliegen.

9. Konjugat aus humanem Albumin (HA) und 2-(4-isothiocyanatobenzyl)-1,4,7,10-tetraazacyclododecan-1,4,7,10-Tetraessigsäure (p-SCN-Bn-DOTA), das in wässrigem Medium bei pH 3-8,5 unlöslich ist und dessen IR-Spektrum eine Absorption bei etwa 700 cm$^{-1}$ zeigt, welches durch ein Verfahren gemäß einem der Ansprüche 1 bis 8 erhältlich ist.

10. Komplex bestehend aus einem Konjugat von HA und p-SCN-Bn-DOTA (HAC) gemäß Anspruch 9 und einem radioaktiven Isotop eines Elementes, ausgewählt aus der Gruppe bestehend aus Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe und Cu.

11. Komplex gemäß Anspruch 10, wobei das radioaktive Isotop ausgewählt ist aus der Gruppe bestehend aus $^{111}$In, $^{177}$Lu, $^{90}$Y, $^{67}$Cu, $^{64}$Cu und radioaktiven Lanthaniden und vorzugsweise $^{177}$Lu ist.

12. Komplex gemäß einem der Ansprüche 10 und 11 für die therapeutische Anwendung.

13. Komplex gemäß einem der Ansprüche 10 und 11 für die Verwendung in der in-vivo-Lokalisierung von Radionukleiden in Organen und Gewebe des menschlichen Körpers für diagnostische und therapeutische Zwecke.

14. Komplex gemäß einem der Ansprüche 10 und 11 für die Verwendung bei der Lokalisierung von mammalen neoplastischen Läsionen durch das R.O.L.L. ("Radioguided Occult Lesion Localization")-Verfahren.

15. Verfahren zum Zubereiten eines HAC-Komplexes gemäß einem der Ansprüche 10 und 11, umfassend die Schritte:

   - Dispergieren eines Konjugats gemäß Anspruch 9 in einem wässrigen Puffer bei pH 4-6;
   - Hinzufügen einer wasserlöslichen Komponente enthaltend ein Ion eines radioaktiven Isotops eines Elements ausgewählt aus der Gruppe bestehend aus Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe und Cu, und
   - Erhitzen unter Rühren auf eine Temperatur von 30-90°C während 20-40 Minuten.

16. Verfahren gemäß Anspruch 15, wobei die wasserlösliche Komponente $^{177}$LuCl$_3$ ist.

17. Verfahren gemäß einem der Ansprüche 15 oder 16, wobei der wässrige Puffer ein Essigsäure/Acetat-Puffer ist, vorzugsweise 1M mit einem pH von 5.

**Revendications**

1. Procédé de fabrication d'un conjugué d'albumine humaine (HA) et d'acide 2-(4-isothiocyanatobenzyl)-1,4,7,10-tétraazacyclododécane-1,4,7,10-tétraacétique (p-SCN-Bn-DOTA), comprenant les étapes consistant :

   a) à faire réagir HA avec p-SCN-Bn-DOTA dans un milieu aqueux à une température comprise entre 35 °C et 45 °C, formant ainsi une suspension contenant un phase solide en suspension dans une phase liquide ;
   b) à séparer la phase solide, contenant le conjugué, de la phase liquide, contenant éventuellement du p-SCN-Bn-DOTA n'ayant pas réagi.

2. Procédé selon la revendication 1, dans lequel l'étape de séparation b) consiste en une étape de filtration ou d'ultrafiltration.

3. Procédé selon la revendication 2, dans lequel un solide obtenu lors de l'étape de filtration ou, respectivement, un rétentat obtenu lors de l'étape d'ultrafiltration, est soumis à une lyophilisation.

4. Procédé selon l'une quelconque des revendications 1 à 3, dans lequel la réaction entre HA et p-SCN-Bn-DOTA est effectuée à une température comprise entre 38 et 42 °C pendant 4 à 24 h, de préférence pendant 8 à 21 h.

5. Procédé selon l'une quelconque des revendications 1 à 4, dans lequel le milieu aqueux est une solution aqueuse ayant une force ionique comprise entre 0,1 et 0,5 M, en particulier une solution de NaCl à 0,9%, éventuellement tamponnée à un pH compris entre 4 et 8,5.

6. Procédé selon l'une quelconque des revendications 2 à 5, dans lequel un seuil de coupure à 10 kDa est utilisé dans l'étape d'ultrafiltration.

7. Procédé selon l'une quelconque des revendications 1 à 6, dans lequel le rapport molaire entre p-SCN-Bn-DOTA et HA est compris entre 100:1 et 1:1, de préférence entre 30:1 et 16:1.

8. Procédé selon l'une quelconque des revendications 1 à 7, dans lequel la HA qui réagit avec p-SCN-Bn-DOTA durant l'étape a) a été préalablement purifiée par ultrafiltration ou par dialyse avec une membrane ayant un seuil de coupure à 10 kDa, en présence d'une solution ayant une force ionique de 0,1 à 0,5 M, afin d'éliminer les stabilisants thermiques présents dans la HA disponible dans le commerce, en particulier le caprylate de sodium et le tryptophanate d'acétyle de sodium.

9. Conjugué d'albumine humaine (HA) et d'acide 2-(4-isothiocyanatobenzyl)-1,4,7,10-tétraazacyclododécane-1,4,7,10-tétraacétique (p-SCN-Bn-DOTA), qui est insoluble en milieu aqueux à un pH compris entre 3 et 8,5 et dont le spectre IR présente une absorption à environ 700 cm$^{-1}$, obtenu par le procédé selon l'une quelconque des revendications 1 à 8.

10. Complexe consistant en un conjugué de HA et de p-SCN-Bn-DOTA (HAC) selon la revendication 9 et un isotope radioactif d'un élément choisi dans le groupe constitué par Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe et Cu.

11. Complexe selon la revendication 10, dans lequel l'isotope radioactif est choisi dans le groupe consistant en $^{111}$In, $^{177}$Lu, $^{90}$Y, $^{67}$Cu, $^{64}$Cu et les lanthanides radioactifs, et est de préférence du $^{177}$Lu.

12. Complexe selon l'une quelconque des revendications 10 et 11 pour un usage thérapeutique.

13. Complexe selon l'une quelconque des revendications 10 et 11 destiné à être utilisé dans la localisation *in vivo* de radionucléides dans les organes et les tissus du corps humain à des fins de diagnostique ou de thérapie.

14. Complexe selon l'une quelconque des revendications 10 et 11 destiné à être utilisé dans la localisation de lésions néoplasiques chez les mammifères par la méthodologie R.O.L.L. (« localisation radioguidée de lésions occultes »).

15. Procédé de préparation d'un complexe HAC selon l'une quelconque des revendications 10 et 11, comprenant les étapes consistant

    - à disperser un conjugué selon la revendication 9 dans un tampon aqueux à un pH compris entre 4 et 6 ;
    - à ajouter un composé hydrosoluble contenant un ion d'un isotope radioactif d'un élément choisi dans le groupe constitué par Sr, Rh, Pd, Sm, Er, Au, Bi, In, Lu, Y, Ce, Pr, Nd, Pm, Sa, Eu, Gd, Tb, Dy, Ho, Tm, Yb, Ga, Ni, Co, Fe et Cu, et
    - à chauffer sous agitation à une température comprise entre 30 et 90 °C pendant 20 à 40 minutes.

16. Procédé selon la revendication 15, dans lequel le composé hydrosoluble est du $^{177}$LuCl$_3$.

17. Procédé selon l'une quelconque des revendications 15 ou 16, dans lequel le tampon aqueux est un tampon acide acétique/acétate, de préférence 1 M avec un pH de 5.

Fig. 1

Fig. 2

Fig. 2A

Fig. 3

Fig. 4

Fig. 5

Fig. 5A

Fig. 5B

Fig. 6

L: lenght

Fig. 7

Fig. 8

Fig. 9

ile:100616 campioni Y Caviglioli Acquired: 6/16/2010 3:40:10 PM
Owner: User1 Subject : AS 1mCi/mg 111 In
T1 - Lane Group

Lane #1
Background Subtraction Regions=377.142 DLU/mm2

| ID | RF | Gross DLU | Background Subtract | Net DLU | Net % Sum |
|---|---|---|---|---|---|
| 1 - Prof | 0.2 | 10,185,352.8 | 70,097.7 | 10,115,255.2 | 89.0 |
| 2 - Prof | 0.5 | 248,715.2 | 44,395.2 | 204,320.0 | 1.8 |
| 3 - Prof | 0.8 | 679,762.4 | 46,147.6 | 633,614.8 | 5.6 |
| 4 - Prof | 1.1 | 429,258.3 | 53,157.4 | 376,100.9 | 3.3 |
| 5 - Prof | 1.6 | 128,034.6 | 96,384.3 | 31,650.3 | 0.3 |
| 1-b - Prof | | 4,673.2 | | | |
| Lane | | 11,846,117.3 | 411,823.7 | 11,434,293.6 | |
| UnRes | | 174,994.0 | 101,641.6 | 73,352.4 | |

Lane #2 - Background Subtraction Regions = 307.196 DLU/mm2

| ID | RF | Gross DLU | Background Subtract | Net DLU | Net % Sum |
|---|---|---|---|---|---|
| 1 - Prof | 0.3 | 6,699,984.1 | 64,710.1 | 6,635,274.0 | 88.3 |
| 2 - Prof | 0.6 | 154,757.4 | 29,976.0 | 124,781.4 | 1.7 |
| 3 - Prof | 0.9 | 484,781.7 | 40,443.8 | 444,337.9 | 5.9 |
| 4 - Prof | 1.2 | 329,516.0 | 50,435.8 | 279,080.1 | 3.7 |
| 5 - Prof | 1.8 | 121,706.2 | 89,452.3 | 32,253.9 | 0.4 |
| 1-b - Prof | | 3,806.5 | | | |
| Lane | | 7,896,063.4 | 335,446.0 | 7,560,617.4 | |

Fig. 10

Lane #1
Background Subtraction Regions= 262.894 DLU /mm2

| ID | RF | Gross DLU | Background Subtract | Net DLU | Net % Sum |
|---|---|---|---|---|---|
| 1 - Prof | 0.0 | 4,039,072.2 | 41,126.2 | 4,047,945.9 | 87.1 |
| 2 - Prof | 0.3 | 51,613.5 | 18,730.8 | 32,882.8 | 0.7 |
| 3 - Prof | 0.5 | 450,315.3 | 43,162.2 | 407,153.1 | 8.8 |
| 4 - Prof | 0.9 | 205,306.6 | 43,976.6 | 161,330.0 | 3.5 |
| 1-b - Prof | | 580.9 | | | |
| Lane | | 4,990,908.2 | 287,069.3 | 4,703,838.9 | |
| UnRes | | 194,600.7 | 140,073.5 | 54,527.1 | |

Lane #2 - Background Subtraction Regions = 704.587 DLU /mm2

| ID | RF | Gross DLU | Background Subtract | Net DLU | Net % Sum |
|---|---|---|---|---|---|
| 1 - Prof | 0.0 | 14,289,209.3 | 145,145.5 | 14,144,063.9 | 88.6 |
| 2 - Prof | 0.4 | 171,442.2 | 69,844.4 | 101,597.7 | 0.6 |
| 3 - Prof | 0.6 | 1,260,910.8 | 99,310.1 | 1,161,600.7 | 7.3 |
| 4 - Prof | 0.8 | 673,345.7 | 118,953.8 | 554,391.8 | 3.5 |
| 1-b - Prof | | 1,556.8 | | | |
| Lane | | 16,655,172.1 | 769,380.2 | 15,885,791.9 | |
| UnRes | | 260,264.2 | 336,126.4 | -75,862.2 | |

Fig. 11

Lane #1

Background Subtraction: Regions = 244.885 DLU /mm2

| ID | RF | Gross DLU | Background Subtract | Net DLU | Net % Sum |
|---|---|---|---|---|---|
| 1 - Prof | 0.1 | 6,058,707.5 | 35,260.9 | 6,023,446.6 | 92.3 |
| 2 - Prof | 0.9 | 620,660.0 | 118,376.0 | 502,284.0 | 7.7 |
| 1-b - Prof | | 3,148.3 | | | |
| Lane | | 6,853,539.1 | 221,954.9 | 6,631,584.2 | |
| UnRes | | 174,171.6 | 68,318.0 | 105,853.5 | |

Fig. 12

Fig. 13

Fig. 14

All tracks @ 280 nm

Fig. 15

All tracks @ 280 nm

Fig. 16

Animal No. 1 - after 43 h                    Animal No. 2 after 38 h

Fig. 17

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4024233 A
- US 4094965 A
- US 6730286 B
- US 20030059368 A

### Non-patent literature cited in the description

- **PAGANELLI G. ; LUINI A. ; VERONESI U. et al.** Use of technetium-99m-labeled colloid albumin for preoperative and intraoperative localization of nonpalpable breast lesions. *Journal of the American College of Surgeons,* 2000, vol. 190 (6), 692-699
- **PAGANELLI G. ; VERONESI U.** Innovation in early breast cancer surgery: radioguided occult lesion localization and sentinel node biopsy. *Nucl Med Commun,* 2002, vol. 23 (7), 625-627
- **VAN DER PLOEG C. et al.** Radioguided occult lesion localisation' (ROLL) for nonpalpable breast lesions: A review of the relevant literature. *EJSO,* 2008, vol. 34 (1), 1-5
- **POVOSKI P. et al.** A comprehensive overview of radioguided surgery using gamma detection probe technology. *World J Surg Oncol,* 2009, vol. 7
- **BOWEN et al.** Preparation of Technetium-99m Human Serum Albumin Macroaggregates. *Am J of Hospital Pharm.,* 1969, vol. 26, 529-534
- **WATANABE N. et al.** *Journal of Nuclear Medicine,* 1997, vol. 38 (10), 1590-1592
- **PATIL.** *Drug Dev. Res.,* 2003, vol. 58, 219-247
- **SCHILLER et al.** *Nuclear Medicine and Biology,* 2008, vol. 35 (2), 227-232
- **JAKUBOWSKI et al.** *J. Anal. At Spectrom.,* 2008, vol. 23 (11), 1497-1507
- **FOWLER J. C. et al.** *Acta Oncologica (Stockholm),* 2007, vol. 46 (1), 105-110
- **B. WESSELS.** Radionuclide selection and model absorbed dose calculations for radiolabelled tumour associated antibodies. *R. Rogus. Med Phys.,* 1984, vol. 11 (5), 638-645
- Metallic radionuclides for radioimmunotherapy. **A.R. FRITZBERG ; C.F. MEARES.** Cancer radioimmunotherapy: Present and Future. Harwood Academic Publishers, 1999, 57-79
- **MCMENAMY et al.** *JBC,* 1958, vol. 233 (6), 1436-1446
- **RITCHI et al.** A spectroscopic study of thiourea derivatives-(III): Infrared and Raman spectra of NN'-disubstituted thioureas Spectrochimica Acta. *Molecular Spectroscopy,* 1971, vol. 27 (9), 1597-1608
- **DADACHOVA E. et al.** Spectrophotometric method for determination of bifunctional macrocyclic ligands in macrocyclic ligand-protein conjugates. *Nuclear Medicine and Biology,* 1999, vol. 26 (8), 977-982
- **MATARRESE et al.** *Applied Radiation and Isotopes,* 2010, vol. 68, 5-13